# EUROPEAN PATENT SPECIFICATION

(11) **EP 1 419 248 B1**
(45) Date of publication and mention of the grant of the patent: **16.03.2005**
(21) Application number: 03747925.0
(22) Date of filing: 22.08.2003
(51) Int. Cl.: C12N 15/10

(54) **METHOD FOR THE SELECTIVE COMBINATORIAL RANDOMIZATION OF POLYNUCLEOTIDES**
VERFAHREN ZUR SELEKTIVEN ZUFALLSMUTAGENESE VON POLYNUKLEOTIDEN
PROCEDE DE REPARTITION ALEATOIRE COMBINATOIRE ET SELECTIVE DE POLYNUCLEOTIDES

(30) Priority: 22.08.2002 US 405650 P; 23.08.2002 EP 02018855
(43) Date of publication of application: 19.05.2004
(73) Proprietor: Direvo Biotech AG, 50829 Köln (DE)
(72) Inventor: KOLTERMANN, André, cc/DIREVO BIOTECH AG, 50829 Köln (DE); KETTLING, Ulrich, cc/DIREVO BIOTECH AG, 50829 Köln (DE); PILLING, Jens, cc/DIREVO BIOTECH AG, 50829 Köln (DE); SPANGENBERG, Oliver, cc/DIREVO BIOTECH AG, 50829 Köln (DE)
(74) Representative: Helbing, Jörg, Dr. Dipl.-Chem.
(86) International application number: PCT/EP2003/009340
(87) International publication number: WO 2004/018674

(56) References cited:
- WO-A-02/46396
- REETZ M T: "Directed evolution of selective enzymes and hybrid catalysts" TETRAHEDRON, ELSEVIER SCIENCE PUBLISHERS, AMSTERDAM, NL, vol. 58, no. 32, 5 August 2002 (2002-08-05), pages 6595-6602, XP004374099 ISSN: 0040-4020
- KUCHNER AND F H ARNOLD A: "Directed evolution of enzyme catalysts" TRENDS IN BIOTECHNOLOGY, ELSEVIER, AMSTERDAM,, GB, vol. 15, no. 12, December 1997 (1997-12), pages 523-530, XP002133872 ISSN: 0167-7799
- WURST H ET AL: "MUTANT PROFILES OF SELECTABLE GENETIC ELEMENTS" PROCEEDINGS OF THE NATIONAL ACADEMY OF SCIENCES OF THE UNITED STATES, vol. 88, no. 22, 1991, pages 9909-9913, XP002226883 1991 ISSN: 0027-8424

## Description

The present invention provides a method for the selective combinatorial randomization (SCR) of polynucleotides at specific sites which comprises providing a double stranded polynucleotide sequence having at least one differing site and selectively randomizing the polynucleotide at or in the proximity to the differing sites without the need for a determination of the sequence position of the differing site.

### Background of the Invention

The basic concept of genetic engineering is the identification of a gene of interest in nature, followed by the transfer of this gene to a production organism and the production of the corresponding gene product - be it an enzyme, an antibody or a secondary metabolite - by fermentation. Heterologous gene expression has been an époque-making step for a simple reason - gene products of enormous value became available in quantities that were far from reach by extraction from natural sources. However, nature certainly did not evolve molecules to serve as a biopharmaceutical, as an industrial enzyme or as a biocatalyst for chemical processes. Therefore, it became very early obvious that the quantitative improvement could be multiplied by a qualitative improvement. Qualitative improvement means modifying the properties or the composition of one or several gene products of interest, with the aim to improve their technical or medical applicability. If the gene products are proteins, e.g. enzymes or antibodies, this qualitative improvement has been termed protein engineering. Other applications have been termed analogously. For example, when dealing with metabolites, the process has been called metabolic engineering. The improvement of bacterial strains has been called strain engineering, etc. Today, there is an increasing demand for such engineering technologies, allowing to engineer gene products to become new functional ingredients in nutrients or consumer products, new catalysts for the chemical industry, or new drugs to target diseases that are not or not sufficiently treatable yet.

Independently of the nature of the gene product of interest, engineering to improve the quality of this gene product relies on the modification of the gene sequence or polynucleotide that encodes it. A wide variety of techniques for the modification of gene sequences are known. In general, one has to distinguish between methods for the generation of new combinations of existing sequence parts on the one hand and methods for the generation of new sequences by mutagenesis on the other hand. Both classes of techniques can further be classified into deterministic and random techniques. While deterministic methods have the aim to generate one or a few polynucleotides with specific sequences, random techniques, on the other hand, have the aim to generate polynucleotides with at least partially random sequences. See Table 1 for a general overview on techniques for the modification of gene sequences.

**Table 1:**

| Techniques for the modification of gene sequences | | |
|---|---|---|
| | Deterministic: | Random: |
| Generation of new combinations of sequences | Insertion or joining together of specific sequences (more generally known as recombinant DNA technology) | Random recombination -homologous or heterologous - of sequence parts (DNA shuffling, RCR (i. e. a method according to WO 01/34835), Step, Itchy) |
| Generation of new sequences by mutagenesis | Defined exchange of one or more nucleotides (known as site-specific mutagenesis, e.g. Kunkel method, etc.) | Random mutagenesis (mutagenic PCR, cassette mutagenesis, method of the invention) |

Techniques for the deterministic generation of new combinations of sequence parts insert a specific sequence into another sequence at a specific site or, more generally, join two or more specific sequences in a specific order together. Insertion or joining is traditionally done by cutting sequences at specific sites with restriction enzymes and ligating the resulting pieces together by means of ligase enzymes. Alternatively, recently developed techniques use recombinase enzymes for the same purpose. These techniques are generally known as recombinant DNA technology. Random recombination techniques, on the other hand, combine sequence parts at more or less randomly chosen positions, i.e. generate in principle all possible combinations of sequences that are provided. This can either be done homologously, i.e. by joining analogous sequence parts from different source sequences, or heterologously, i.e. by joining non-analogous sequence parts from different source sequences. Random recombination methods known in prior art are exemplarily DNA shuffling (Stemmer, Nature 379:389, 1994), RCR (recombination method as disclosed in WO 01/34835), Step (Staggered extension process, Zhao et al., Nat. Biotechnol. 16(3):258, 1998), Itchy (Incremental Truncation for creation of Hybrid Enzymes, Lutz et al., PNAS. 98(20):1248, 2001). WO 02/46396 discloses a further approach for recombination by applying mismatch repair enzymes correcting nucleotide mismatches in the preceding generated heteroduplexes.

Techniques for the deterministic generation of new sequences change one or more nucleotides at specific sites of a polynucleotide for a different nucleotide. Although being specific with regard to the resulting polynucleotide sequence - and not only with regard to the site of the exchange - these methods are traditionally called site-specific mutagenesis methods. A well-known technique enabling the defined exchange of a specific nucleotide to be chosen in a polynucleotide is the protocol according to Kunkel (PNAS, 82(2):488, 1985). Techniques for the random generation of new sequences, on the other hand, lead to pools of polynucleotides with sequences that are not determined. With regard to the position, this randomization of nucleotides can either be done again randomly over the whole gene sequence, e.g. by modified PCR protocols, or at defined positions or regions, e.g. by exchanging sequence parts with their randomized counterparts.

In general, deterministic techniques have the aim to generate one or a few desired sequences. These gene sequences are either known or expected to lead to improved gene products. Accordingly, deterministic techniques rely either on the knowledge or on the theoretical modeling of the relation between genotypes and phenotypes of gene sequences. Random techniques do not require knowledge of the relation between genotypes and phenotypes of gene sequences, but instead rely on methods for the efficient identification of gene sequences with a desired phenotype out of the pool of random sequences that are generated.

There exists a simple relation between the degree of modification of a gene sequence and the intended improvement factor of the gene product: the higher the intended improvement factor is, the more modifications of the gene sequence are usually required. Random recombination techniques are limited in this respect, since these techniques do not generate new sequences but only recombine existing ones. Techniques for the random generation of new sequences, i.e. random mutagenesis techniques, are therefore of enormous importance, since only these techniques allow the introduction of new variety and thereby the generation of new sequences that are not existent in nature yet.

Random mutagenesis techniques either introduce random mutations homogeneously over the entire target sequence, or enable the localization of the randomization to discrete positions or regions of the polynucleotide of interest.

Most methods for homogeneous randomization of entire target sequences work by increasing the frequency of misincorporations during polynucleotide amplification.

Lehtovaara and coworkers (Lehtovaara, P.M. et al., Protein Eng. 2(1): 63, 1988) describe a method for introducing all types of base substitution mutations randomly into a nucleic acid. The method comprises the extension of a primer hybridized to the nucleic acid to be mutagenized in four separate reactions - one for each nucleotide - to generate a population of molecules, each copied from the template and terminating at all possible positions of the particular nucleotide; misincorporation of nucleotides at the variable 3' ends generated before; and completion of the molecules to forms that can be amplified and cloned.

Cadwell and Joyce (PCR Methods Appl., 2(1):28, 1992; PCR Methods Appl. 3(6):136, 1994) describe a random mutagenesis technique referred to as mutagenic PCR. The modified polymerase chain reaction is performed under conditions that reduce the fidelity of nucleotide incorporation during DNA synthesis by using unequal concentrations of the four dNTPs and adding manganese instead of magnesium ions.

Virnekas et al. (Nucleic Acids Res. 22(25):5600, 1994) describe a random mutagenesis technique that uses trinucleotide phosphoramidites. These trinucleotide represent codons for all 20 amino acids, and are used as reagents for the chemical synthesis of mutagenized oligonucleotides.

Besides these techniques for homogeneous random mutagenesis of nucleic acids, there are several methods published for the selective randomization of specific sites of a polynucleotide sequence.

Wells et al. (Gene 34(2-3):315, 1985) describe a method for the randomization of a sequence of interest at specific sites or regions. The method uses mutagenic oligodeoxynucleotide cassettes to generate random nucleotide substitutions. The introduction of a DNA cassette allows saturation of a target amino acid codon with multiple mutations. This procedure of complete randomization of the amino-acid sequence of interest and re-introduction into the gene as a cassette is also described by Loeb et al. (Genome 31(1):112, 1989) and Oliphant et al. (Gene 44(2-3):177, 1986). The approach of oligonucleotide-cassette mutagenesis as region-specific random mutagenesis targeted to a particular set of amino acids is known in the literature (Kuchner and Arnold, TIBTECH 15:523, 1997).

US Patent 5,723,323 (1985) discloses a method for saturation mutagenesis at specific sites in a sequence by use of synthetic polynucleotide coupling. The resulting, stochastically generated polynucleotide sequences are subsequently introduced into vectors containing the gene of interest.
In a particular mode of carrying out this process, stochastic genes are produced by stochastic copolymerization of the four kinds of deoxyphosphonucleotides, A, C, G and T from the two ends of an initially linearized expression vector, followed by formation of cohesive ends in such a fashion as to form a stochastic first strand of DNA constituted by a molecule of expression vector possessing two stochastic sequences whose 3' ends are complementary, followed by the synthesis of the second strand of the stochastic DNA.

Hermes et al. (Gene 84(1):143, 1989; Proc. Natl. Acad. Sci. USA 87(2):696, 1990) describe a method to randomize larger parts of a gene by use of so-called "spiked" oligodeoxyribonucleotide primers. The method was developed for the random mutagenesis of the gene for triosephosphate isomerase. By providing oligonucleotides containing a certain percentage of the non-matching bases at every position, a library of mutants was produced with the mutations restricted to those sequence parts that are defined by the primer binding sites.

Lanio and Jeltsch (Biotechniques 25(6):958, 1998) describe another approach with mutagenic primer oligonucleotides to randomize selected parts of a gene with the wildtype being excluded from the transformants. With the mutagenized site being used as the cloning site, modified clones can efficiently be isolated after the mutagenesis step.

Reetz et al. (Tetrahedron 58:6595, 2002) describe an approach for the engineering of enantioselective enzymes with a first step comprising random mutagenesis over the entire length of the enzyme, screening for improved variants and subsequent sequence determination and thereby identification of so called "hot spots" or "hot regions", as positions within the enzyme potentially responsible for improved enantioselectivity. Second, at such "hot spots" or "hot regions" saturation mutagenesis or cassette mutagenesis is specifically applied. The method requires sequence determination and identification of the positions to be mutagenised prior to the introduction of mutations.

In summary, all the above-mentioned random mutagenesis methods can be classified by their requirement for sequence information. A first set of methods is directed to randomization of polynucleotides that comprise entire genes, genomes or parts of genes, and, therefore, do not require the underlying sequence information. However, these methods do not teach any possibility of introducing mutations limited to sites that are relevant or essential for the function or phenotype of the gene product encoded by the polynucleotide or that have been arbitrarily selected by the experimentator. A second set of methods, on the other hand, are directed to randomization of particular sites in a polynucleotide sequence. These methods range from randomization of single, specific positions to the randomization of entire regions. All these methods do, however, require knowledge of the sequence information at the site to be mutagenized. This sequence information is then, for example, used to synthesize mutagenic primers that bind at these sites, or to synthesize oligonucleotide cassettes with a definable degree of mutations to be inserted at these sites by use of restriction enzymes that cut specifically at or next to these sites. Also, these methods are not useful if several sites separated from each other in a polynucleotide sequence are to be randomized simultaneously, if the sites to be randomized are not fixed but change during a set of engineering experiments, or if there is no efficient possibility do determine the sequence of the target polynucleotides and to identify therein explicitly the relevant or essential sites.

It would, therefore, be advantageous to have a random mutagenesis method that enables the efficient randomization of sites without the requirement for sequence information on the target polynucleotides. It would be particularly advantageous to have a random mutagenesis method that enables the randomization of relevant or essential sites within a target polynucleotide without the requirement for prior explicit identification of these sites. Relevant or essential sites in a polynucleotide are easily and efficiently identified by comparison of two or more polynucleotides and selection of the sites at which these two or more polynucleotides differ. Therefore, it would be particularly advantageous to have a random mutagenesis method that enables the randomization of sites at or in proximity to those positions at which two or more polynucleotide sequences differ from each other without the need for a determination of the sequence position of the differing site. Methods with the aforementioned characteristics have not heretofore been available.

### Summary of the Invention

The technical problem underlying the present invention is to provide a method that enables the efficient randomization of sites without the requirement for sequence information on the target polynucleotides. A particular aspect of the technical problem underlying the present invention is to provide a method for the selective randomization of polynucleotides at *relevant or essential sites* without requiring the explicit knowledge of these sites. This technical problem has been solved by the embodiments of the present invention.

Therefore, the present invention is directed to a method for the randomization of polynucleotides at relevant or essential sites. These sites are defined by positions at which two or more polynucleotides differ from each other. The randomization provides polynucleotide populations that encode a diversity of phenotypes, whereby the diversity is restricted to relevant or essential sites or to the proximity of relevant or essential sites. The method comprises the steps providing polynucleotides that differ at one or more sites from each other, whereby these differing sites define the sites that are to be randomized;
generating heteroduplices from the at least one polynucleotide;
recognizing the resulting differing site(s);
selectively randomizing the polynucleotides at or in proximity to these differing sites. The method does not need a sequence analysis, i.e. a determination of the sequence position of the sites to be randomized, prior to randomization.

Furthermore, the present invention is directed to a method for altering polynucleotide characteristics by combination of the randomization of polynucleotides as described above with the selection or screening of these polynucleotides or of the corresponding gene products. The invention is also directed to a method for altering polynucleotide characteristics by combination of the randomization of polynucleotides as described above with other random mutagenesis techniques such as mutagenic PCR or cassette mutagenesis and/or with in-vitro recombination techniques such as the method disclosed in WO 01/34835 and/or with the selection or screening of these polynucleotides or of the corresponding gene products.

In a first aspect of the invention, the method is directed to saturation mutagenesis of polynucleotides at positions that are characterized by mutations in an original polynucleotide sequence, whereby these mutations are generated in a preceding process that comprises subjecting the original polynucleotide to a homogeneous random mutagenesis method and selecting or screening those polynucleotide variants that have desired characteristics. Homogeneous random mutagenesis techniques typically have a bias toward a subset of all possible mutations. Accordingly, a combination of homogeneous random mutagenesis techniques with selection or screening steps can result in the selection of mutations that are only partially optimal for the gene product. When making use of the invention according to the first aspect, these *pre-selected positions* can be randomized completely, i.e. any of the naturally occurring nucleotide is introduced at these positions, thereby enabling to select from the resulting focused library with high efficiency variants with the optimal mutation.

In a second aspect of the invention, the method is directed to randomization of polynucleotides at regions that are characterized by mutations in these regions in an original polynucleotide sequence, whereby the mutations are generated in a preceding process that comprises subjecting the original polynucleotide to a homogeneous random mutagenesis method and selecting or screening those polynucleotide variants that have desired characteristics. When intending to engineer polypeptides by means of random mutagenesis techniques there is often the problem, that these mutagenesis techniques only exchange single nucleotides while the mutagenesis of one amino acid to any other amino acid to a certain extent requires the exchange of two or even three nucleotides in the particular codon. However, the probability of exchanging two or even three nucleotides in a particular codon by means of homogeneous random mutagenesis techniques is relatively low. When making use of the invention according to the second aspect, regions that can be identified as being relevant by identification of at least partially improving mutations in these regions via a pre-selection step are randomized specifically, thereby enabling to select from the resulting focussed library with high efficiency variants with the optimal mutation. These regions can have a size of a codon, i.e. three nucleotides, or can be larger, up to 30 or more nucleotides.

In a third aspect of the invention, the method is directed to randomization of polynucleotides at sites that correspond to codons in a polypeptide that have been screened for being tolerant to the exchange for codons encoding a specific amino acid. When intending to engineer polypeptides by means of random mutagenesis techniques there is often the problem, that a significant fraction of the randomized polynucleotides have no function at all, for example because the particular amino acid residue is necessary for the structure or for the folding mechanism of the polypeptide. When making use of the invention according to the third aspect, codons that can be identified as being exchangeable can selectively be randomized. For example, after every codon in a polynucleotide is exchanged for nucleotides coding for an alanine, all variants still encoding functional polypeptides are used as the starting polynucleotides in step (i) of the method of the invention as described above. This decreases the complexity to be screened significantly, thereby increasing the efficiency of engineering polypeptides by means of random mutagenesis drastically.

In a fourth aspect of the invention, the method is directed to randomization of polynucleotides at sites at which naturally occurring polynucleotides differ from each other. Analogous or related genes from the same or from different species are often highly homologous, having sometimes more than 90% homology at the nucleotide level. When making use of the invention according to the fourth aspect, polynucleotide populations can efficiently be generated where the mutagenesis is restricted to those sites at which such homologous genes are different, without determination of the sequence of these naturally occurring, homologous genes.

In a fifth aspect of the invention, the method is directed to the efficient randomization of polynucleotides at several, pre-defined sites simultaneously. It has been a significant problem to generate populations of polynucleotides being randomized at several regions or positions that are distributed over a large sequence such as a gene encoding a polypeptide, an operon encoding a metabolic pathway, or an entire genome. When making use of the invention according to the fifth aspect, regions that are known as being relevant can efficiently be randomized by providing in step (i) as described above two or more polynucleotides whose sequences differ at these particular sites from each other. For example, two or more immunglobulin-encoding polynucleotides are provided that have the same sequence and differ only in the complementarity-determining regions (CDRs) of the heavy and the light chain, leading to a population of polynucleotides that are randomized specifically at the CDRs.
The following detailed description describes the preferred features, advantages and the utility of the present invention. The following drawings are provided in order to explain further the present invention in supplement to the detailed description:

### Brief Description of the Drawings

Figure 1 depicts schematically and exemplarily the method of the invention.
Figure 2 shows a first embodiment of the invention, wherein a single position is randomized.
Figure 3 shows a second embodiment of the invention wherein several nucleotides are removed in 3' direction.
Figure 4 shows a third embodiment of the invention, wherein regions are randomized at and in proximity in both directions to the differing site.
Figure 5 shows electropherograms of polynucleotides subjected to the treatment with CEL I, MutY, TDG and Endonuclease IV
Figure 6 shows the results of dITP incorporation
Figure 7 shows the results of the amplification of IMP-containing templates

### Detailed Description of the Invention

In the framework of this invention the following terms and definitions are used. The term "polynucleotide" corresponds to any genetic material of any length and any sequence, comprising single-stranded and double-stranded DNA and RNA molecules, including regulatory elements, structural genes, groups of genes, plasmids, whole genomes, and fragments thereof. The term "site" in a polynucleotide refers to a certain position or region in the sequence of the polynucleotide. The term "position" in a polynucleotide refers to specific single bases in the sequence of the polynucleotide. The term "region" in a polynucleotide refers to stretches of several bases in the sequence of the polynucleotide. The term "differing site" is defined as at least one nucleotide which do not form a A/T or G/C Watson-Crick base pairing. The term "polypeptide" comprises proteins such as enzymes, antibodies and the like, medium-length polypeptides such as peptide inhibitors, cytokines and the like, as well as short peptides down to a amino acid sequence length below ten, such as peptidic receptor ligands, peptide hormones, and the like. The term "gene product" corresponds to any product, including, but not being limited to, polypeptides, that is encoded by a polynucleotide and that has a particular phenotype being selectable by any means of screening or selection technique.

The term "relevant or essential site(s)" or "pre-defined site(s)" refers to positions at which two or more polynucleotides differ from each other but without those positions necessarily being identified by any kind of sequence analysis.

The phrase "with no sequence related determination needed" in accordance with the invention means that a determination of the sequence position of the differing sites is not required prior to randomization.

The term "pre-selection step" describes an optional step preceding the method of the invention, whereby polynucleotide variants resulting from a homogenous randomization mutagenesis method are subjected to selection or screening of variants for any desired characteristics.
Therefore, the term "pre-selected position(s)" describes "relevant or essential sites" obtained by the aforementioned step.

The terms "random mutagenesis" or "randomization" as used in this description indicate the manipulation of polynucleotides by unpredicted, stochastical replacements of the original nucleotide at a position with any other nucleotide. Alternatively, the term can also indicate the manipulation of polypeptide sequences by unpredicted, stochastical replacements of the original amino acid residue at a position with any other amino acid residue. Randomization or random mutagenesis methods usually lead to populations of polynucleotides or polypeptides that are related but differ from each other in one or more positions. "Heteroduplices" refer to double-stranded polynucleotide molecules comprised of single strands that differ at one or more positions from each other. If two single-stranded polynucleotides that differ in one or more positions are annealed, the resulting double stranded heteroduplex comprises base-paired and non base-paired regions. In DNA, adenine (A) usually pairs with thymidine (T) and guanine (G) usually pairs with cytosine (C). All other combinations usually do not form base-pairs and are therefore termed "mismatches". "Nicks" are incisions in the backbone of a double-stranded polynucleotide in one of either strands. These single-stranded breaks can be generated by an agent that is able to introduce nicks into a double-stranded polynucleotide. "Nucleobases" or "bases" are abbreviated as given in Table 2.

**Table 2:**

| **Abbreviation** | **Nucleobase** |
|---|---|
| A | Adenine |
| C | Cytosine |
| G | Guanine |
| T | Thymidine |
| U | Uracile |
| I | Inosine |
| N | A, C, G, T, or U |
| V | Universal bases |
| - | AP site or abasic site (position with the base being removed from the backbone) |
| X | Mutation (position at which two or more polynucleotides differ from each other) |

The term "universal base" refers to base analogs that are able to pair with more than one of the naturally occurring bases. Analogously, the term "universal nucleotide" refers to nucleotide analogs that can be incorporated into polynucleotides and after incorporation are able to pair with more than one of the naturally occurring nucleotides.

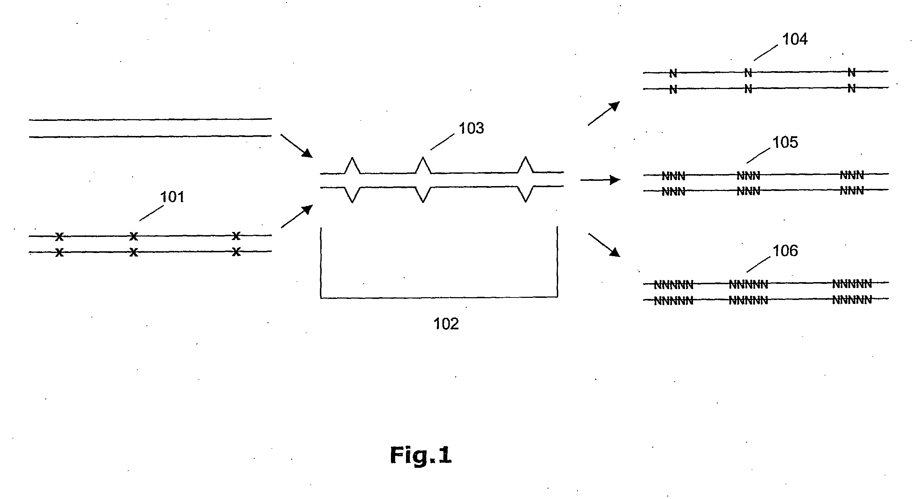

The principle of the present invention is schematically and exemplarily shown in Fig. 1. The method is directed to the randomization of polynucleotides at relevant or essential sites. These sites are defined by positions at which two or more polynucleotides differ from each other. The randomization provides polynucleotide populations that encode a diversity of phenotypes, whereby the diversity is restricted to relevant or essential sites or to the proximity of these sites. The method comprises the provision of polynucleotides that differ at one or more sites from each other (101, mutations indicated with an "X"), the generation of heteroduplices from these polynucleotides (102), and the recognition and selective randomization of the resulting mismatches (103, randomized positions indicated with an "N"), either focused to a single mismatching nucleotide (104), or to a codon of three nucleotides (105), or to a region or a larger stretch of surrounding nucleotides (106).

In a preferred embodiment, the method comprises the following steps providing polynucleotides that differ at one or more sites from each other, whereby these one or more differing sites define the sites that are to be randomized;
generating heteroduplices from the polynucleotides provided in step (a) leading to mismatches at the one or more sites;
introducing single-strand nicks at one or more of the mismatches generated in step (b), by means of an agent that is able to specifically recognize mismatch sites;
removing one or more nucleotides from the polynucleotide heteroduplex starting at the single-strand nicks generated in step (c);
filling the one or more gaps produced in step (d) under conditions that lead to the incorporation of one or more mismatching nucleotides, thereby randomizing the polynucleotides specifically at relevant or essential sites.

In a particularly preferred embodiment, steps (c) and (d) are executed simultaneously, i.e. mismatching nucleotides are removed directly in one step. Alternatively, the nucleobase of the mismatching nucleotide is removed simultaneously with the introduction of the single-strand break, thereby leading to an apurinic /apyrimidinic (AP) site (abasic site), which is afterwards modified to lead to an extendable 3'-OH end. In another particularly preferred embodiment, this single nucleotide gap is extended further 5'-3', 3'-5' or in both directions simultaneously. In another particularly preferred embodiment, the filling of the gap according to step (e) leads to a nick at the end of the polymerized stretch of nucleotides, which is then covalently closed by means of a ligase enzyme, optionally in combination with a polynucleotide kinase. In another particularly preferred embodiment, there is no gap formed, but instead steps (d) and (e) are executed simultaneously, i.e. nucleotides next to the nick introduced in step (c) are removed simultaneously to the incorporation of one or more mismatching nucleotides. The remaining nick is preferably covalently closed by means of a ligase enzyme. As an alternative, after incorporation of one or more mismatching nucleotides, the polymerization conditions are switched to non-mutagenic conditions, and the strand is synthesized without incorporation of mismatching nucleotides.

Starting material for the method of the invention are two or more polynucleotides that differ at one or more sites from each other. These differences mark the sites where randomization is performed. These polynucleotides are preferably provided as linear PCR products, either in a single-stranded or in double-stranded form. Alternatively, other linear polynucleotides, such as linearized plasmids or parts of a gene can be used analogously. When starting with two polynucleotides, these polynucleotides are preferably provided in a single-stranded form, one as the plus and one as the minus strand, thereby enabling the selective generation of heteroduplices. When starting with more than two polynucleotides, these polynucleotides are preferably provided in a double-stranded form in order to allow every possible heteroduplex pair be formed. The fraction of homoduplices, that per definition do not contribute to the further random mutagenesis process, decreases when increasing the number of double-stranded polynucleotides provided. For example, if two polynucleotides are provided at the same concentrations, the fraction of homoduplices is on average 50%, whereas, if twenty polynucleotides , are provided at equal concentrations, the fraction of homoduplices is on average 5%.

The polynucleotides provided in step (a) can originate from different sources. They can originate from the preceding randomization of an original polynucleotide combined with one or more selection or screening steps that select those polynucleotides that encode gene products with improved characteristics. Preferably, the preceding randomization is done homogeneously, leading to mutations over the entire polynucleotide. Furthermore, starting polynucleotides can originate from the scanning of an original polynucleotide for sites - comprising single positions or longer regions - that are tolerant for a nucleotide exchange in the polynucleotide and/or for an amino acid exchange in the encoded polypeptide. Alternatively, starting polynucleotides are analogous or related genes or parts thereof isolated from the same or different species, showing a minimum degree of homology. As a further alternative, the positions at which polynucleotides differ can be introduced arbitrarily in order to provide marked polynucleotides to be selectively and efficiently randomized at these positions. The polynucleotide can have a length in the range between a few nucleotides and up to several kilobases. Preferably, polynucleotides are between 10 and 100,000 nucleotides long, more preferably between 100 and 10,000 nucleotides, and most preferably between 500 and 5,000 nucleotides.

In step (b), heteroduplices are generated from the polynucleotides provided in step (a). If the starting materials are double-stranded polynucleotides, the polynucleotides are mixed, then subjected to conditions that lead to melting of the double-strands to produce single-stranded molecules, which is followed by reannealing of these single strandes (Current Protocols in Molecular Biology, 1987-1988, Wiley Interscience). If the starting materials are single-stranded polynucleotides, those are mixed and randomly annealed to form double-stranded polynucleotides. The resulting heteroduplex molecules comprise mismatches, which can selectively be targeted by chemical, biochemical and/or enzymatic means.

In step (c), nicks are introduced into the heteroduplices specifically at or directly next to the mismatch sites. Such a nick is either a sole single-strand break in the phosphodiester backbone at the 5' or 3' side of the mismatch site, or the removal of the entire mismatching nucleotide, or the removal of several nucleotides at or around the particular mismatch site. The introduction of nicks is usually random with respect to the particular strand in the heteroduplex to be nicked. In particular embodiments, however, one of the two strands can be selectively nicked, thereby increasing the possible frequency of randomized sites per polynucleotide in the resulting populations.

Single-strand breaks at mismatch positions can be produced by several enzymatic and non-enzymatic ways. Vsr endonuclease from *E. coli* is particularly useful. The enzyme cleaves double-stranded DNA at T:G base-pair mismatches and produces a single-strand break 5' to the incorrectly paired T with a free 3'-OH and a 5' -phosphate residue at this nick. The enzyme shows a preference for T:G mismatches within a particular sequence context. The consensus sequence is N₁ T^{A}/_{T}GN₂ N stands for A, T, G or C, the underlined T is opposed by a dG base (Gläsner, W. et *al.,* J. Mol. Biol. 245(1):1, 1995; Lieb, M. and Rehmat, S., J. Bacteriol. 177(3):660, 1995). Another useful enzyme is the *E. coli* endonuclease IV. This enzyme is a class II AP endonuclease with 3'-repair phosphodiesterase activity cleaves the phosphodiester backbone on the 5' side of the apurinic/apyrimidinic (AP) sites leaving a 5'-terminal 2-deoxyribose 5-phosphate residue (dRP, removable by dRPase activity) and a free 3' -OH residue. The enzyme removes 3' blocking fragments, e.g. phosphoglycoaldehyde, deoxyribose-5-phosphate, 4-hydroxy-2-pentenal, and phosphate groups from the 3'ends of DNA left by AP lyase activity (Friedberg, E.C. et al., DNA Repair and Mutagenesis, ASM Press, Washington: 157-158, 1995; Levin, J.D. *et al.,* J. Biol. Chem. 266(34):22893, 1991). *E. coli* Endonuclease V (deoxyinosine 3'-endonuclease) is another useful enzyme. It recognizes mismatches in duplex DNA and cleaves the second and third phosphodiester bonds 3' to the mismatch at 95% and 5% frequency, respectively. The enzyme produces a nick with 3'-hydroxyl and 5'-phosphoryl groups in the strand with the mismatch closest to the 5'end. Unlike the members of the glycosylase-class of enzymes endonuclease V does not appear to release free bases from DNA. Another particularly useful enzyme is Endonuclease V, which cleaves DNA duplexes containing AP sites, urea residues, hairpin or unpaired loops, flaps, and pseudo-Y structures. (Yao, M. *et al.,* J. Biol. Chem., 269(23):16260, 1994). The mode of action of the enzyme depends on the reaction conditions, i.e. pH, presence of MnCl₂ or MgCl₂. A further enzyme performing incision on the 3'- side of the mismatch site in one of the two DNA strands in a heteroduplex with a broad specificity for different mismatches is the CEL I-like nuclease ("CEL-1") isolated from celery (Oleykowski et al., Nucleic Acids Res. 26(20):4597, 1998).
A further, particularly useful enzyme in this context is MutY. The enyzme is a bifunctional glycosylase. It recognizes A/G and A/8-oxo-dG mismatches in duplex DNA and cleaves the strand containing the A. The opposite strand is not cleaved. MutY has an associated AP lyase activity (Lu, A.L. and Hsu, I.C., Genomics 14(2):249, 1992; Friedberg, E.C. *et al.,* DNA Repair and Mutagenesis, ASM Press, Washington: 157-158, 1995). MUG from E. coli is a further useful enzyme. MUG removes pyrimidines uracil (deamination of cytosine) and thymine (deamination of 5-methylcytosine) from U/G and T/G mismatches (Barrett, T.E. *et al.,* Cell 92(1):117, 1998; Barrett, T.E et *al*., EMBO. J. 18(23):6599, 1999). TDG (Thymine mismatch DNA glycosylase, from M. thermoautotrophicum) is another particularly useful enzyme. TDG recognizes T/G (U/G, G/G, T/T, T/C) mismatches (deamination of 5'-methylcytosine to thymine) in dsDNA. TDG is a monofunctional glycosylase. The enzyme specifically removes thymine and uracil bases mispaired with guanine through hydrolysis of their N-glycosidic bond, thereby generating abasic sites in DNA. A further useful enzyme is Human endonuclease IV homolog APE/HAP1. The enzyme cleaves DNA at AP sites forming nicks in DNA (Yacoub, A. *et al.,* Cancer Res. 57(24):5457, 1997; Duguid, J.R. *et al.,* Cancer Res. 55(24):6097, 1995). In contrast to endonuclease IV, APE1 shows only weak 3'-repair diesterase activity on deoxyribose fragments located at DNA strand breaks Demple, B and Harrison, L., Annu. Rev. Biochem. 63:915, 1994; Xu, Y.J. et *al.,* J. Biol. Chem. 273(44):28837, 1998). A further useful enzyme is E. coli exonuclease III. This enzyme has a class III AP endonuclease activity besides the 3'- to 5'-exonuclease activity. It acts on 3'-OH, 3'-phosphate, and 3'-phosphoglycolate groups (Friedberg, E.C. *et al.,* DNA Repair and Mutagenesis, ASM Press, Washington: 157-158, 1995).

As an alternative to enzymatic processes, single-strand breaks at mismatch positions can also be produced by chemical cleavage (CMC-chemical mismatch cleavage). Osmium tetroxide and hydroxylamine known of their application in "mutant profiling" for the detection of mismatched base pairs (Wurst, H. et al. Proc. Natl. Acad. Sci. USA. 88: 9909, 1991) are examples of suitable chemicals. Osmiumtetroxide, potassium permanganate is known to recognise and modify a range of mismatched bases (T/C, T/G, T/T and C/T, C/A, C/C mismatches). Potassium permanganate/ tetraethylammonium chloride and hydroxylamine are next to others further alternatives (Roberts, E*. et al.,* Nucleic. Acids. Res. 25(16):3377, 1997).

In a further embodiment and as an alternative to introducing single-strand nicks in step (c), only the nucleobase of a mismatching nucleotide is removed, thereby generating an abasic site at the mismatch position but without incision of the strand. Examples of useful agents for the removal of nucleobases at mismatch positions are DNA glycosylases having no AP lyase function, e.g. UDG (from E. coli). According to this embodiment, step (d), i.e. the removal of nucleotides in the incised strand to generate a gap, can be avoided. The randomization as described in step (e) is done by polymerization using the abasic site-containing strand as a template, thereby leading to the incorporation of nucleotides other than the nucleotide at or next to the mismatch position in the original polynucleotide. Therefore, the generation of an abasic site at a mismatch position is analogous to the incorporation of a universal nucleotide after introduction of a single-strand nick at a mismatch position.

The removal of single-strands according to step (d) can be limited to several nucleotides to generate single-strand regions in proximity to the mismatch positions within the double-stranded polynucleotides. Alternatively, the removal of the single-strands according to step (d) can be unrestricted, thereby extending the gap from the mismatch positions to the end of the polynucleotides. Exonucleases and polymerases can be advantageously used for this purpose. Examples of useful exonucleases are Lambda-exonuclease (5'→ 3' exonuclease) (Little, Gene Amplification & Analysis 2, 135-145 (1981); T7 exonuclease (5'→3'exonuclease), T5 D15 exonuclease (5'→3' exonuclease, Sayers et al., J. Biol. Chem. 265:18311-18317, 1990), 5'-3' exonuclease from the bacteriophage N4 (Guinta et al., J. Biol. Chem. 261:10736-10743, 1986), 5'-3'-exonuclease from nuclear extracts (Exol) from Saccharomyces cerevisiae (Huang and Symington, Mol. Cell. Biol., 3125-3134, 1993), Exonuclease III (3'→5'exonuclease), Exonuclease I (3'→5' exonuclease) (Brody et al., J. Biol. Chem. 261:7136-7143, 1986; Brody and Doherty, Biochemistry 24:2072-2076, 1985), YNT20 from Saccharomyces cerevisiae (3'-5' exonuclease) (Hanekamp and Thorsness, Current Genetics 34:438-448, 1999), DNA-polymerase-lilsubunit-epsilon of E. coli (3'→5' exonuclease) (Krutyakov, Mol. Biol. 32:197-199, 1998), Examples of useful polymerases are DNA polymerase I (5'→3' polymerase, 3'→5' and 5'→3'exonuclease) (Rigby et al., J. Mol. Biol. 113 :237-251, 1997), Taq (Tth) polymerase (5'→3' polymerase, 3'→5' and 5'→3'exonuclease) (Longley M.J. et al., Nucleic Acids Res. 18(24):7317-22, 1990), Klenow fragment (5'→3' polymerase, 3'→5' exonuclease) (Sanger, Proc. Natl. Acad. Sci. USA 74:5463-5467, 1977), T4 DNA polymerase (5'→3'polymerase, 3'→5' exonuclease) (Young et al., Biochemistry 31(37):8675, 1992), Pwo, Pfu, Pfx, Tub, Vent, Tma, UITma polymerases (5'→3'polymerase, 3'→5'exonuclease, Newton and Graham, in: PCR, Spektrum Akad. Verlag Heidelberg, 1, 1994).

The filling of the gaps according to step (e) is carried out by polymerization of nucleotides. Preferably, the filling can be done with a standard polymerase under conditions that lead to an increased frequency of misincorporations (e.g. conditions of mutagenic PCR as described by Cadwell, R.C and Joyce, G.F., PCR Methods Appl. 2(1):28, 1992; PCR Methods Appl. 3(6):136, 1994). More preferably, the filling of the gaps can be carried out with a polymerase and universal nucleotides. Universal nucleotides are characterized as being able to form basepairs alternatively with two or more of the four standard nucleobases; Therefore, universal nucleosides are, but not limited to, dI (2'-deoxy-inosine), dP (P coding for 6H,8H-3,4-dihydropyrimido[4,5-c][1,2]oxazin-7-one, with "p" serving as pyrimidine (C or T) analogue, Lin and Brown, Nucleic Acids Res, 17(24):10373-83, 1989), dK (K coding for N6-methoxy-2,6-diaminopurine, with "K" serving as a purine (G or A) analogue, Lin and Brown, Nucleic Acids Res. 20(19):5149-52, 1992). Further, as universal bases can be used 3-nitropyrrole (Nichols et al., Nature 369:492, 1994; Bergstrom et al., J. Am. Chem. Soc. 117: 1201, 1995) or 4-, 5-, and 6- nitroindole (Loakes et al, Nucleic Acids Res. 22(20):4039-43, 1994).
Alternatively, the filling of the gaps according to step (e) can be carried out with a polymerase and unequal mixtures of the four standard nucleotides (dATP, dCTP, dGTP, dTTP). As a further alternative, filling of the gaps can be carried out with a polymerase in four separate reactions, whereby in each reaction one of the four standard nucleotides (dATP, dCTP, dGTP, dTTP) is lacking. Furthermore, filling of the gaps can be carried out with a polymerase and a mixture of standard (dATP, dCTP, dGTP, dTTP) and universal nucleotides such as dITP. Dependent on the incorporation rate of each of the nucleotides, mixtures of unequal concentrations of each nucleotide are provided. For example, in order to enforce the integration of a nucleotide with lower incorporation efficiency compared to others, this nucleotide is provided in higher concentration.

In a further alternative, a variant of a "split-mix" approach is performed. Therein, filling of gaps is carried out in separate reactions, whereby in each reaction only one of the four standard nucleotides (dATP, dCTP, dGTP, dTTP) or one agent of the group of universal nucleotides such as dITP is provided. In a preferred embodiment, filling of the gaps is done in four separate reactions with only one of the four standard nucleotides (dATP, dCTP, dGTP, dTTP) provided in each reaction. If, for example, the gaps generated in step (d) have the length of one nucleotide, every single-nucleotide gap in a polynucleotide molecule is filled with an A if the polynucleotide is present in the first reaction, with a C in the second reaction, with a G in the third reaction, and with a T in the fourth reaction, independently of the template nucleotide. Thereby, the polynucleotide is randomized at the gaps generated in step (d). If, on average, more than one gap is present in a polynucleotide, the resulting polynucleotides are mixed after the polymerization step, then again split into different reactions and subjected to a further polymerization step. Preferably, this is done over several cycles of split and mix. More preferably, between two cycles, the newly generated polynucleotides are subjected to a mismatch recognition, single-strand cleavage and gap generation step (as done in steps (a) - (d)), thereby using the non-original nucleotides introduced in one step as mismatching nucleotides in the following step. As another alternative, filling of the gaps can be carried out with a polymerase and a mixture of random nucleotide trimers, with specific oligonucleotides generated from the original pool of genes but carrying mutations, with completely random oligonucleotides, or with a combination of these.
Further on, the filling of the gaps according to step (e) can be carried out with a ligase and specific and/or random oligonucleotides or mixtures thereof. Instead of modifying the conditions during polymerization, the polymerase can also be chosen to have a high error rate (Suzuki, M. et al., J. Biol. Chem. 272(17):11228, 1997).

Polynucleotides generated in step (e) can be subjected to amplification procedures.
In vitro PCR amplification is performed under conditions offering any of the standard nucleotides dNTPs. Preferably, the amplification is carried out with unequal mixtures of the four standard nucleotides in order to compensate any bias for the nucleotide incorporated opposite to an universal nucleotide during the amplification. Polynucleotides obtained in step (e) can also be amplified *in vivo.*

In a first embodiment of the method of the invention the degradation of the nicked strand according to step (d) is limited to one nucleotide to generate an unpaired nucleotide only at the specific mismatching positions.

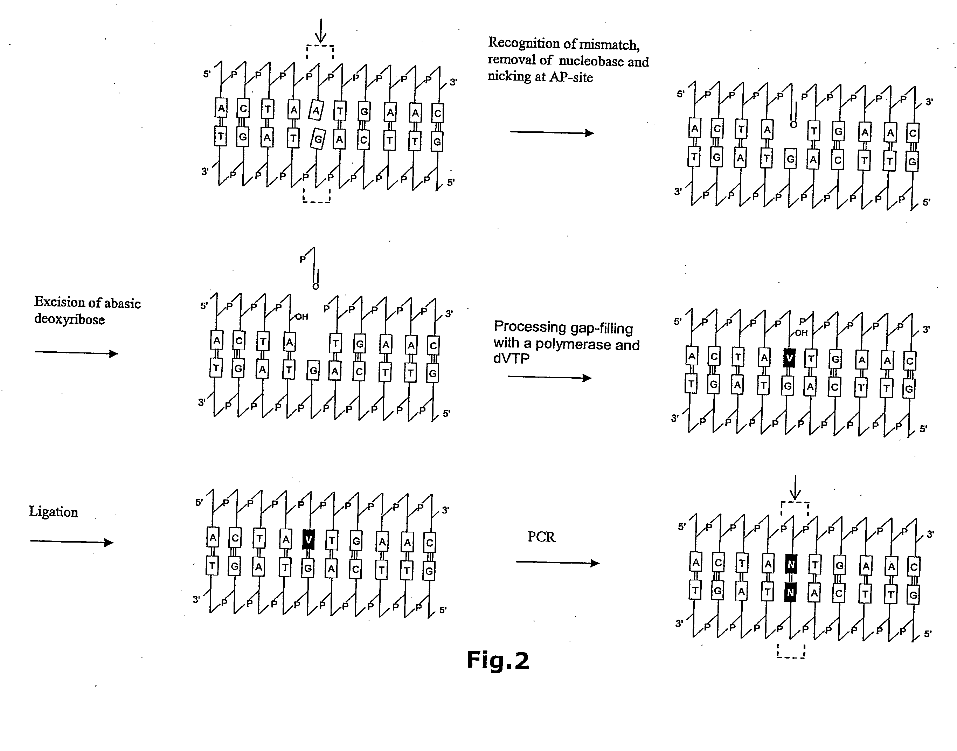

A particularly preferred variant of this embodiment is depicted in Fig. 2. According to this variant, the mismatching nucleobase is first removed by an agent that is able to specifically recognize mismatches and that has DNA glycosylyase and AP lyase activity. The resulting nicked abasic deoxyribose moiety is preferably removed by an agent having AP endonuclease activity such as Endonuclease IV from *E.coli* (Friedberg, E.C. *et al*., DNA Repair and Mutagenesis, ASM Press, Washington:157-158, 1995; Levin, J.D. *et al.,* J. Biol. Chem. 266(34):22893, 1991), human Endonuclease IV (Yacoub, A. *et al.,* Cancer Res. 57(24):5457, 1997; Duguid, J.R. *et al.,* Cancer Res., 55(24):6097, 1995), Exonuclease III from *E.coli* (Friedberg, E.C. *et al.,* DNA Repair and Mutagenesis, ASM Press, Washington:157-158, 1995), leading finally to a single-nucleotide gap having an extendable 3'-OH at the position of the former mismatch. This embodiment can be followed by the introduction of a single, universal nucleotide, such as dITP, by means of a polymerase, and ligation of the resulting nick by means of a ligase enzyme, optionally combined with a polynucleotide kinase.

PCR amplification of this modified polynucleotide or amplification by inserting in a vector and transformation into a cell lead finally to a population of polynucleotide molecules comprising random mutations specifically at the mismatching position.

In a second embodiment of the invention, the removal of nucleotides from the nicked strand according to step (d) is done simultaneously to the incorporation of new nucleotides according to step (e) by means of a polymerase having 5'-3' exonucleolytic activity or strand displacement activity to randomize positions at the 3' side of the mismatching positions.

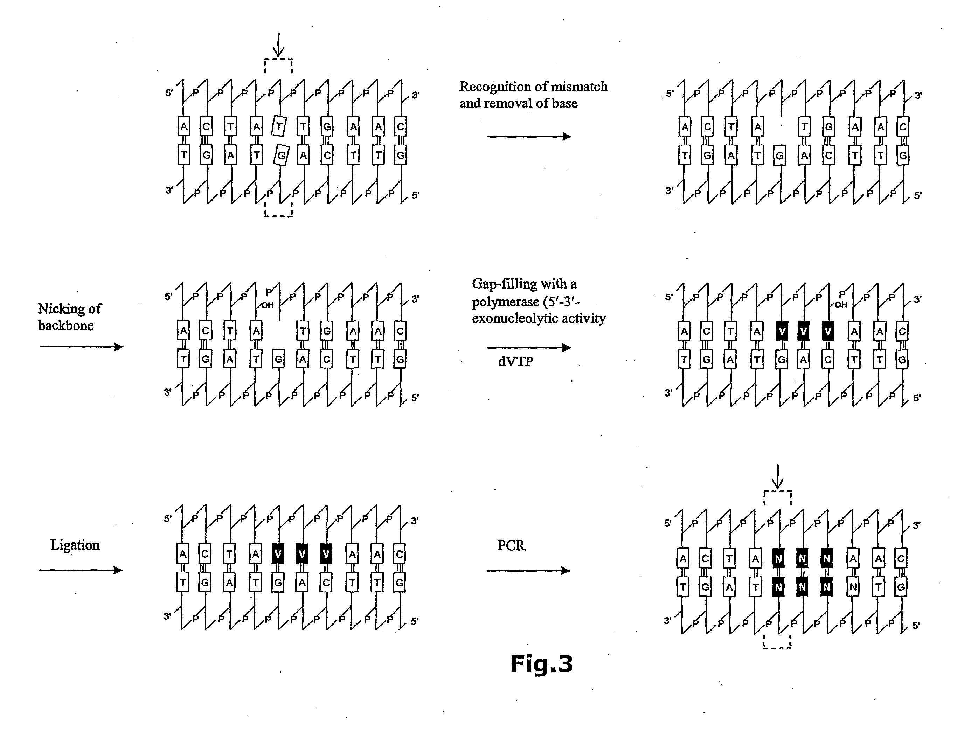

A particularly preferred variant of this second embodiment is depicted in Fig. 3. According to this variant, the mismatching nucleobase is first removed by an agent that is able to recognize mismatches and to excise the corresponding nucleobase resulting in an AP site. Preferably, an enzyme with DNA glycosylase function such as TDG (Thymine mismatch DNA glycosylase from *M. thermoautotrophicum,* Neddermann, P. et al., J. Biol. Chem. 271(22):12767, 1996) or MUG (Mismatch uracil DNA glycosylase from *E.coli,* Barrett, T.E *et al.,* Cell 92(1):117, 1998; Barrett, T.E *et al.,* EMBO. J. 18(23):6599,1999) is used for this step. The phosphodiester bond 5' of the AP site is then hydrolyzed by means of a second agent leading to an extendable 3' OH end. Preferably an enzyme having AP endonuclease function such as E.coli Endonuclease IV (Friedberg, E.C. *et al.,* DNA Repair and Mutagenesis, ASM Press, Washington:157-158, 1995; Levin, J.D. *et al.,* J. Biol. Chem. 266(34):22893, 1991) or human Endonuclease IV (Yacoub, A. *et al.,* Cancer Res. 57(24):5457, 1997; Duguid, J.R. *et al.,* Cancer Res. 55(24):6097, 1995) is used for this step. The resulting 3' OH end is then extended by means of a polymerase optionally having dRPase (deoxyribose phosphatase) function in order to remove the remaining abasic deoxyribose phosphate moiety, as e.g. Human DNA polymerase β (Matsumoto et at., Science 269(5224):699, 1995), *Drosophila* ribosomal protein S3 (Sandigursky et al., J. Biol. Chem. 272(28):17480, 1997). Particularly useful polymerases with 5'-3'-exonucleolytic activity for the removal of nucleotides during the incorporation of new nucleotides are DNA polymerase I (Rigby et al., J. Mol. Biol. 113:237-251, 1977) or Taq polymerase from Thermus aquaticus . Particularly useful polymerases with strand-displacement activity for the removal of nucleotides during the incorporation of new nucleotides are DNA polymerase δ, large fragments of rBst DNA polymerase from B. stearothermophilus, Phi29 DNA polymerase (Giesler et al., Amersham Pharma Biotech). If a polymerase with strand-displacement activity is used the displaced single-strand has to be cleaved by means of a DNase IV or mammalian FEN-1 or Rad27 from *Saccharomyces cerevisiae* (Negritto et al., Molecular and Cellular Biology 21(7):2349, 2001). Incorporated nucleotides are either universal bases (such as dITP, dPTP, dKTP) or standard nucleotides under conditions that lead to an increased misincoporation rate. After ligation of the resulting nick by means of a ligase enzyme, optionally combined with a polynucleotide kinase, the polynucleotides are either PCR-amplified or amplified by inserting into a vector and transformation into a cell lead finally to a population of polynucleotide molecules comprising random mutations specifically 3' downstream from the mismatching position. In another variant of this preferred embodiment randomization can be done by a first polymerization step under conditions that lead to a high frequency of misincorporation and a second polymerization step under conditions that lead to a low frequency of misincorporation. In particular, the first polymerization step is carried out with a polymerase having 5'-3' exonucleolytic activity and using universal nucleotides. The second polymerization step is then carried out with a polymerase having 5'-3' exonucleolytic activity and using standard nucleotides. As an alternative to the aforementioned variants, a polymerase with DRPase but without 5'-3'-exonucleolytic activity and strand-displacement activity can be used. Then only a single nucleotide is incorporated leading to the same result as the first embodiment.

In a third embodiment of the invention, the removal of nucleotides from the nicked strand according to step (d) is done by means of an exonuclease thereby allowing to randomize a region extending from the mismatch site either to the 3' side, or to the 5' side, or to both, the 3' and the 5' side. The size of this region is preferably confined by controlling the exonucleolytic digestion.

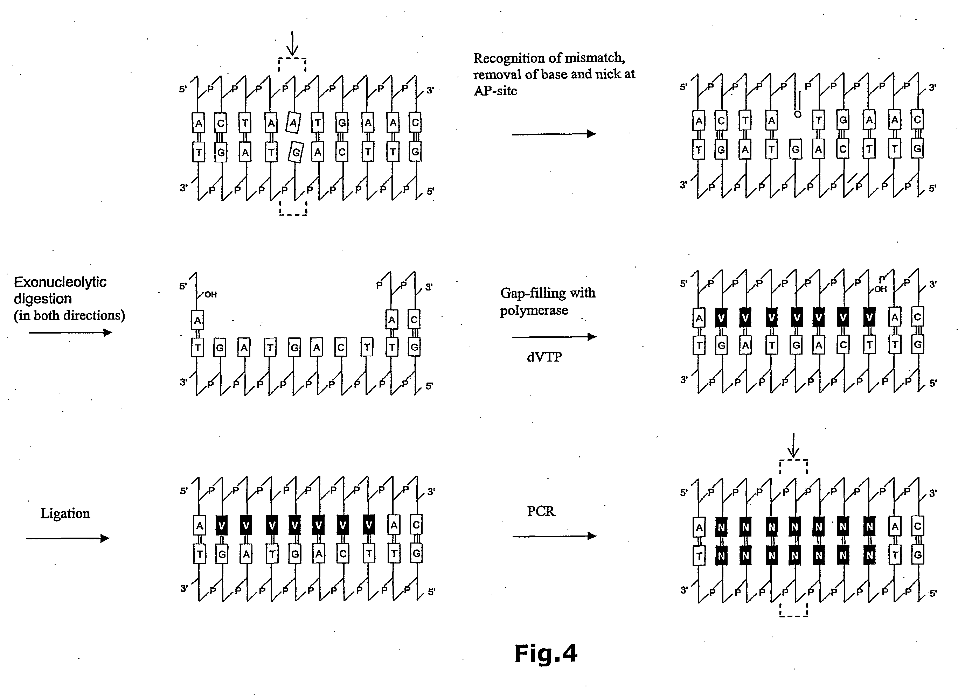

A particularly preferred variant of this third embodiment is depicted in Fig. 4. According to this variant, the mismatching nucleobase is first removed by an agent that is able to specifically recognize mismatches and that has DNA glycosylase and AP lyase activity. The resulting nicked abasic deoxyribose moiety can optionally be removed by an agent having AP endonuclease activity such as E.coli Endonuclease IV (Friedberg *et al., 1995*), Human Endonuclease IV (Yacoub et al., 1997). The nick is then extended to a gap of a certain size by means of an enzyme having exonuclease activity. In a particularly preferred embodiment the gap is extended in 3' direction by means of an exonuclease that specifically has single-strand 3'-5'-exonucleolytic activity such as Exonuclease III from *E.coli* (Friedberg *et al.,* 1995) or *E.coli* Exonuclease I (Brody et al., J. Biol. Chem. 261:7136, 1986). In another particularly preferred embodiment the gap is extended in 5' direction by means of an exonuclease that specifically has single-strand 5'-3'-exonucleolytic activity such as λ -Exonuclease or T7-5'-exonuclease derived from the bacteriophage T7. In a further, particularly preferred embodiment the gap is extended in both directions by means of an exonuclease that has single-strand 3'-5'- and 5'-3'-exonucleolytic activity such as Bal 31 from Alteromonas espejiana (Gray et al., Nucleic Acid Res. 2:1459-1492, 1975) or by means of a blend of enzymes having single-strand 3'-5'-exonucleolytic and 5'-3'-exonucleolytic activity. The resulting 3' OH end is then extended by means of a polymerase lacking 5'-3'-exonucleolytic and strand-displacement activity. Particularly useful polymerases for this purpose are T7 DNA polymerase, Klenow fragement, T4 DNA polymerase. Incorporated nucleotides are either universal bases such as dITP or standard nucleotides under conditions that lead to an increased misincoporation rate.

In another variant of this preferred embodiment randomized oligonucleotides of different length are being hybridized to the gaps of ssDNA generated as outlined above. Optionally, these olignucleotides may contain varying degrees of universal bases.
After ligation of the resulting nick by means of a ligase enzyme, optionally combined with a polynucleotide kinase, the polynucleotides are either PCR-amplified or amplified by inserting into a vector and transformation into a cell leading finally to a population of polynucleotide molecules comprising random mutations specifically 3' downstream or 5' upstream or to both direction from the former mismatching position.

### Experimental Section:

### Example 1: Generation of DNA-Heteroduplices

The following polynucleotides were used to generate double-stranded polynucleotides with homologous and heterologous regions.

The (+) strand of polynucleotide 1 and the (-) strand of polynucleotide 2 as well as the (+) strand of polynucleotide 3 and the (-) strand of polynucleotide 4 were mixed in equimolar amounts to yield a solution of 1 µg DNA in 20 µl water. Annealing was performed by heating the solution in a PCR cycler to 94 °C and subsequent cooling with a rate of 0.04 °C/s to 50 °C. The (+) strand of polynucleotide 1 and the (-) strand of polynucleotide 2 create a double-stranded polynucleotide with a mismatch at position 51 (Heteroduplex 1). The (+) strand of polynucleotide 3 and the (-) strand of polynucleotide 4 create a double-stranded polynucleotide with a variety of mismatches such as T/G, C/T, A/A, G/T, C/A, A/C, A/A at the positions 51, 172, 202, 221, 259, 348, 349 respectively which comprise 3 of the 8 possible mismatch classes (Heteroduplex 2).

### Example 2: Introduction of single-strand nicks at mismatches

Mismatches in the heteroduplices are recognized by DNA-Glycosylases. In this example the following DNA-Glycosylases are used: TDG (Thymin-DNA-Glycosylase, the enzyme recognizes under standard conditions preferably mismatches in the order T/G >> T/C > T/T and cleaves specifically the single-strand at T); MutY (MutY-DNA-Glycosylase, the enzyme recognizes under standard conditions preferably A/G and A/C mismatches and cleaves specifically the single-strand at A). Under non- standard conditions, both enzymes show other preferences.

The analysis of the cleavage reaction was carried out with fluorescent-labeled heteroduplices each strand being labeled at its 5'-end. Fluorescently labeled single-stranded polynucleotides were generated with 5'-end labeled primer and a standard PCR protocol. The respective PCR products were denatured and re-annealed under standard PCR conditions (94 °C → 40 °C, 0.04 °C/s) and purified (QIAgen PCR purification kit). These fluorescence labeled heteroduplices were submitted to enzymatic reactions. The resulting DNA fragments were analysed by polyacrylamide capillary electrophoresis with fluorescence detection.

The addition of 1 µl TDG (2 U/µl, R&D Systems) and 2 µl of 10 x TDG-Buffer (R&D Systems) to 20 µl (1 µg/20 µl) of Heteroduplex 2 and incubation for 1h at 65 °C demonstrated preferred cleavage of T/G and T/T mismatches, under these conditions.
The addition of 1 µl (2 U/µl, R&D Systems) MutY to 0.5 µg of Heteroduplex 2 in 50 µl REC-Buffer (R&D Systems) and subsequent incubation for 2h at 37 °C demonstrated preferred cleavage at MutY for A/G mismatches, under these conditions.

### Example 3: Introduction of single-strand nicks at AP sites

Mismatches in heteroduplices can be recognized and modified by the cleavage of a nucleoside residue at one of the two mismatch basepairs. A double-stranded polynucleotide with an apurinic site (AP site) site can be cleaved by *E. coli* endonuclease IV under the following conditions: The double-stranded polynucleotide substrate with an apurinic site was generated by annealing (94°C → 40°C, 0.04 °C/s) oligonucleotide 1 (5'-GAATATGCAC, AGAGTG[Sp-d]TCC TTATGGC; SEQ ID NO:5; "Sp-d" = abasic site) and oligonucleotide 2 (5'-GCCATAAGGA GCACTCTGTG CATATTC; SEQ ID NO:6). A total of 1 µg annealing product was incubated in 20 µl of TDG Buffer (R&D Systems) with 4 U endonuclease IV (E.coli, MBI Fermentas) for different periods of time. The reaction was stopped by adding 5 µl of 6 x loading buffer (MBI Fermentas) and boiling for 10 min at 95°C. The reaction products were analysed using a 15% polyacrylamide gel and ethidiumbromide staining. There was an increase in intensity of the expected cleavage product with prolonged incubation.

### Example 4: Trimming of 3'-ends for polymerase reaction

Heteroduplex DNA displaying mismatches may be nicked by bi-functional DNA-Glycosylases which subsequent to glycosylase activity further incise at the 3'site via ß-elimination thereby producing an obstructive 3'end. These 3' blocking groups can be removed by *E. coli* endonuclease IV to generate suitable primers for extension reactions. Fragments generated by TDG action (Example 2) that had an obstructive 3'-end were isolated from a denaturing PAGE gel employing standard procedures. In the following the blocked fragments were incubated with endonuclease IV using conditions as outlined in Example 3. The functionality of the trimmed oligonucleotide was demonstrated by primer extension under standard conditions. Reaction products were analysed as outlined in Example 2 and showed the extensibility of the endonuclease IV treated oligonucleotide.

### Example 5: Recognition of mismatch positions with a mixture of CEL 1, TDG, MutY, and Endonuclease IV

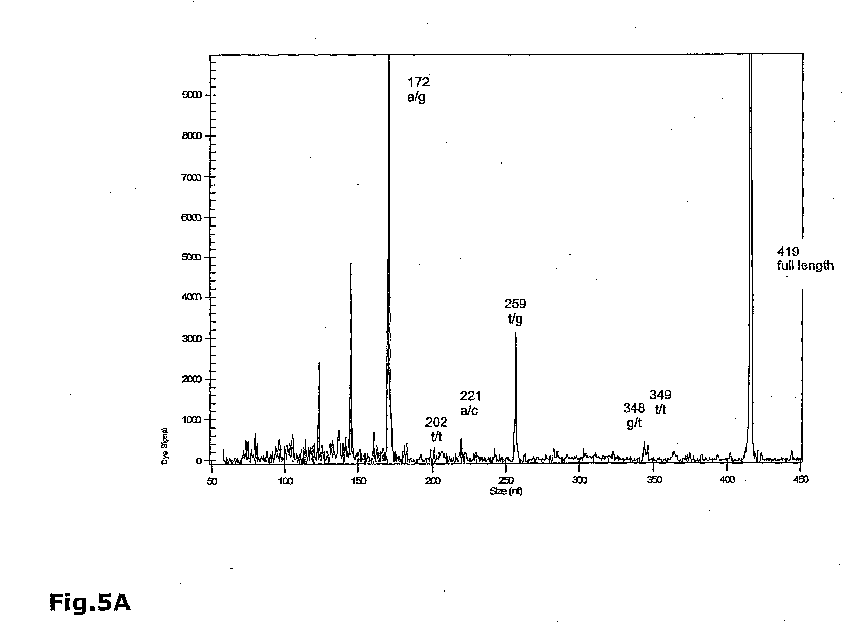

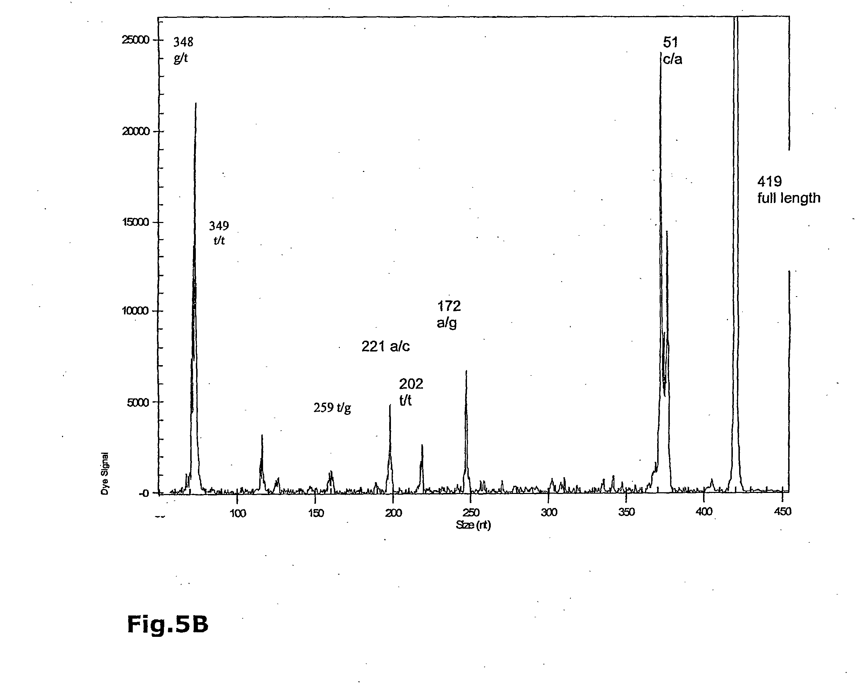

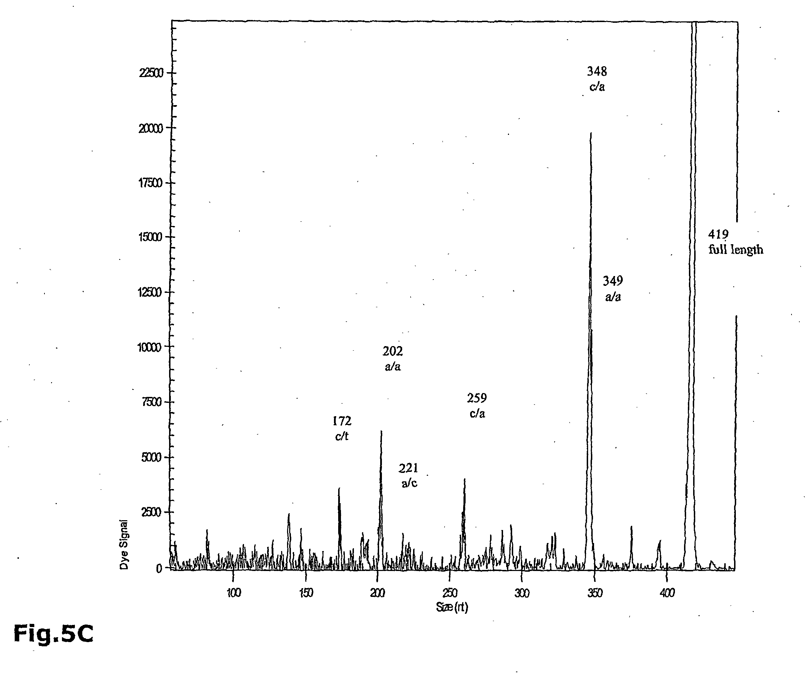

Two separate samples with 3 µg flourescently labeled 419bp-heteroduplex 1 DNA consisting of a (+) strand of polynucleotide 4 (SEQ ID NO:4) and a (-) strand of polynucleotide 3 (SEQ ID NO:3 ) and 3 µg of the fluorescently labeled 419bp heteroduplex 2 DNA consisting of a (+) strand of polynucleotide 3 (SEQ ID NO:3) and a (-) strand of polynucleotide 4 (SEQ ID NO:4) were treated with 25 U CEL1 (Transgenomic, Omaha, NE, USA) for 2 min at 37°C in a reaction volume of 100 µl 20 mM HEPES-KOH, pH 7.4; 10 mM KCI; 3 mM MgCl₂. The reaction was terminated by adding 10 mM EDTA. Further on, 100 µl 10 mM HEPES-KOH, pH 7.4; 100 mM KCI; 10 mM EDTA and 10 U *E. coli* MutY DNA glycosylase (Trevigen, Gaithersburg, MD, USA) were added. After incubation at 37°C for one hour, 10 U human TDG DNA glycosylase (Trevigen, Gaithersburg, MD, USA) were added and the reaction-mix was incubated for an additional hour at 65°C.
Samples were purified using the MinElute PCR Purification Kit (Qiagen, Hilden). To remove the deoxyribose-5-phosphate from the 3'ends at the nicked abasic sites the eluted dsDNA was incubated with 10 U Endonuclease IV (MBI Fermentas) in 80 µl 50 mM Tris-acetat, pH 7.5, 50 mM KCI, 1 mM EDTA, 0.05% Samples were analyzed by polyacrylamide capillary electrophoresis with the results are shown in Figure 5 . Therein the annotations at the peaks refer to the position of recognized mutation within the polynucleotide, obtained by difference of the fragment size to the full length (419 bp) of the polynucleotide. Below a size of 60 nucleotides a detection was not possible, due to instrumental limitations.
Figure 5A and Figure 5B depict the fragments produced from the fluorescently labeled (+) strand and from the fluorescently labeled (-) strand of heteroduplex 1, respectively.
Figure 5C shows the fragments from the fluorescently labeled (+) strand of heteroduplex 2. The (-) strand of heteroduplex 2 was not faceled in this experiment, due to instrumental limitations.

All mismatches in the heteroduplex molecules were recognized as expected although with different efficiencies. Mismatches t/t at the position 202 and 349 in the (-) strand of heteroduplex 1, respectively, (corresponding to positions 217 and 70 in the (+) strand) could be detected only with low efficiency.

### Example 6: Incorporation of dITP

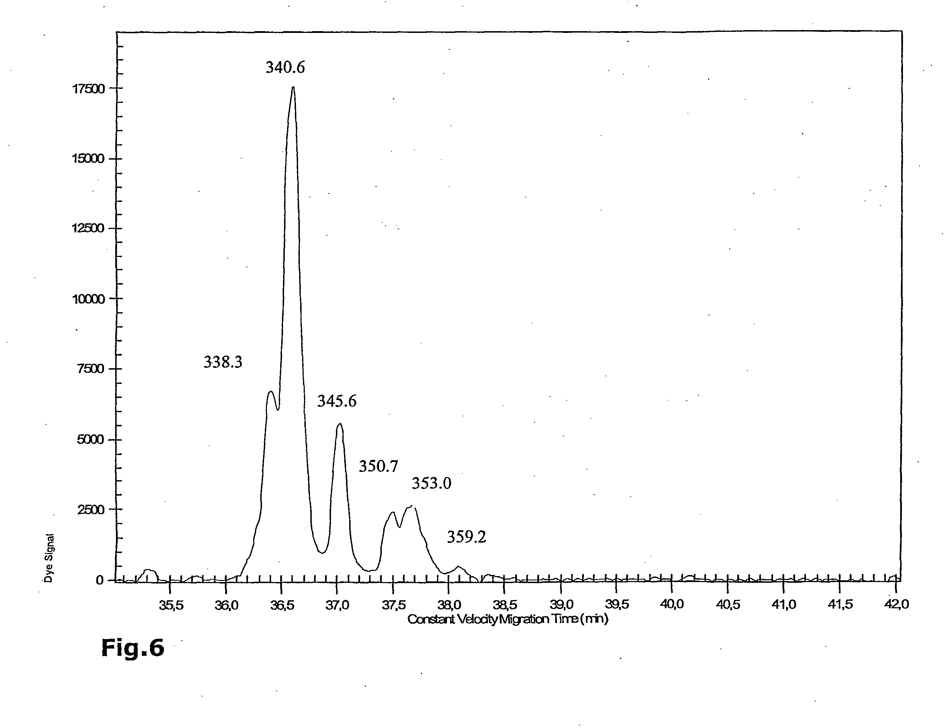

A polynucleotide was generated by digesting fluorescently labeled 909 bp polynucleotide 3 with *Nae*I*.* The 338 bp fragment was purified (QIAgen Minelute PCR product purification kit), melted and annealed (94°C→ 40 °C, 0.04 °C/s) with unlabelled polynucleotide 3 prior to elongation by Taq-DNA-Polymerase. The extension reactions were carried out by addition of 10 µl Buffer (750 mM Tris-HCl, pH 8.8; 200 mM (NH₄)2SO₄; 25 mM MgCl₂; 0.1 % (v/v) Tween® 20) with 0.05 U/µl Taq DNA polymerase (MBI Fermentas)) in 100 µl and by subsequent incubation at 72 °C for 20 min in the absence of dNTPs but in the presence of 2 mM dITP. Elongation products were detected by DNA-fragment analysis described in example 2 under standard and mutagenic conditions demonstrating the incorporation of dIMPs. Figure 6 shows the extension of the 338 bp fragment with dITP. Extension products resulting from incorporation of dITP are indicated. Under the experimental conditions, the majority of the products are extended by two deoxyinosine residues and elongation proceeds up to at least 21 deoxyinosine residues. two deoxyinosine residues and elongation proceeds up to at least 21 deoxyinosine residues.

### Example 7: Randomization by incorporation of dNTPs in four separate reactions according to the split-mix protocol

Filling of nucleotide-gaps was carried out with human DNA polymerase β in four separate reactions, whereby in each reaction only one of the four dNTPs (dATP, dCTP, dGTP, dTTP) was present.
To study the incorporation of mismatching dNTPs at single nucleotide gaps, double stranded DNA molecules each having a single-nucleotide gap were generated by incubating 0,5 pmol of primer 23 (5'-Fluorophor-CGAGCGTTGC ATATGTGGAA GAAGATCATA T; SEQ ID NO:7), 2 pmol of primer 11 (5'-[P]-GCACATGAAT ATGCACAGAG TGTTCCTTAT GGC; SEQ ID NO:8) and 1 pmol template 31 (5'-GCCATAAGGA ACACTCTGTG CATATTCATG TGCXATATGA TCTTCTTCCA CATATGCAAC GCTCG, where X stands for A, T, C or G; SEQ ID NO:9) in 10 µl EB buffer for 5 min at 95 °C and cooling down slowly to 40 °C. Incorporation of dNTPs was carried out with 5 U human DNA polymerase ß (Trevigen) in 20 µl 50 mM Tris-Cl (pH 8.8), 10 mM MgCl₂, 100 mM KCI, 1.0 mM DTT, 10 % glycerol with 5 mM of one of the four dNTPs (dATP, dCTP, dGTP, dTTP). After incubation for 2 min at 37°C the enzyme was removed by extraction with phenol/chloroform and the dsDNA was precipitated with ethanol.
The gap-closing reaction was performed with 10 U E. coli T4 DNA ligase in 20 µl 1x ligase buffer (40 mM Tris-HCl (pH 7.8), 10 mM MgCl₂, 10 mM DTT, 0.5 mM ATP). With the primers shown above heteroduplexes representing all 12 possible single nucleotide mismatches were formed and analyzed. Formation of ligation-products was observed by polyacryl amide capillary electrophoresis. Efficiencies of dNTP incorporation and ligation are shown in table 3.

**Table 3**

| Incorporation of: | A | C | G | T |
|---|---|---|---|---|
| with the template nucleotide being: | | | | |
| T | 67,00% | 50,00% | 29,00% | 41,00% |
| G | 56,00% | 67,00% | 47,00% | 50,00% |
| C | 33,00% | 50,00% | 20,00% | 23,00% |
| A | 55,00% | 60,00% | 50,00% | 86,00% |

### Example 8: Ligation of polynucleotides containing dIMP at the 3'-end

Fluorescently labeled oligonucleotide 3 (5'-Fluorophore-CGAGCGTTGC ATATGTGGAA GAAGATCATA TI; SEQ ID NO:10) with a dIMP at the 3'-end was mixed with oligonucleotide 4 (5'[P]-GCACATGAAT ATGCACAGAG TGTTCCTTAT GGC; SEQ ID NO:11) and unlabeled oligonucleotide 3. After denaturation and annealing (94°C → 50°C, 0.04 °C/s), the oligonucleotides were ligated using 25 U T4-DNA-Ligase (MBI Fermentas) overnight at 16 °C under standard conditions. Ligation products of 65 nt single-stranded oligonucleotides were detected using the DNA-fragment analysis described in example 2.

### Example 9: Amplification of templates containing dIMP stretches

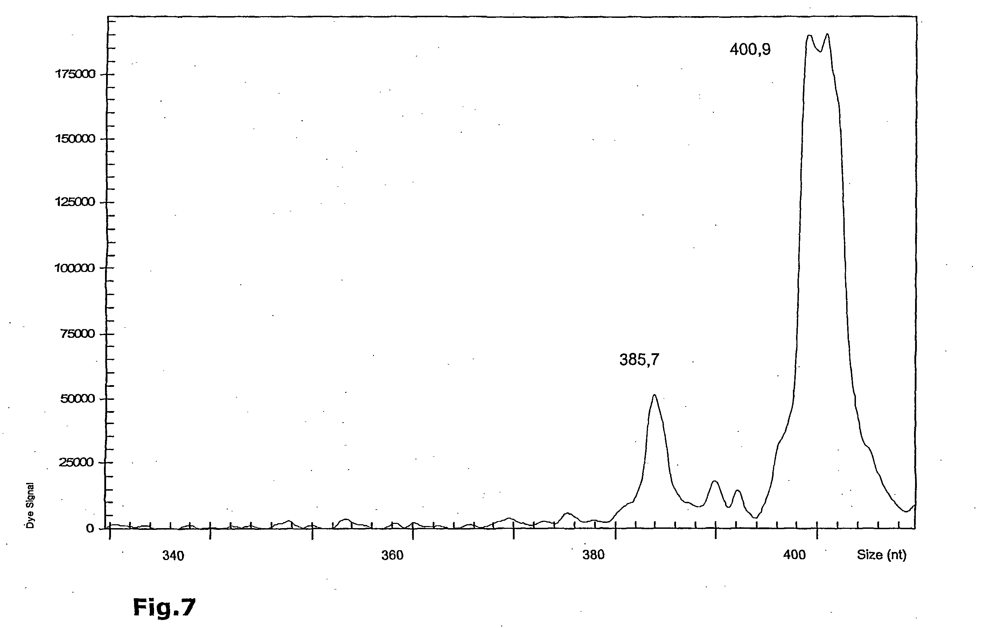

Standard PCR was performed using 100 pmol of primer 1 (5'-GATCATATTG CACTGCATAT GCACAG-3'; SEQ ID NO:12) and 100 pmol of primer 2 (5'-Fluorophor-CAAGGCTCAT GTTGATAACA TC-3'; SEQ ID NO:13) 10 µl 750 mM Tris-HCl, pH 8.8; 200 mM (NH₄)₂SO₄; 0,1 % (v/v) Tween® -20, 10 fmol template vector carrying the subtilisin wt gene , 200 µM dNTPs, 5 U Taq DNA polymerase (MBI Fermentas), ad 100 µl aqua dest. The following cycler protocol was used: 1' 94 °C, 25 cycles of 1' 94 °C, 1' 55 °C, 1.5' 72 °C, one cycle of 6' 72 °C. The dominant peak at 400 bp in Figure 7 indicates that more than 90% of the amplification product is full-length. In less than 10% a shorter fragment of 385bp in length was generated.

### Example 10: Randomization of a subtilisin gene at specific positions

The wild type gene of subtilisin E (SEQ ID NO:14; apre gene from B. subtilis) and a variant thereof (SEQ ID NO:15; a mutant identified by random mutagenesis and subsequent screening for improved activity) were employed in order to generate variants of the subtilisin gene that were randomized at those positions that differ between these two sequences.

Linear polynucleotides were generated by PCR amplification. Two plasmids, each containing one of the two genes were used as templates. Primer L (5'-CGTTGCATAT GTGGAAGAAG ATC-3'; SEQ ID NO:16) and primer R (5'-GAAGCAGGTA TGGAGGAAC-3'; SEQ ID NO:17) were used as primers. Reaction conditions: 10 µl 200 mM Tris-HCl, pH 8.8; 100 mM KCl; 100 mM (NH₄)₂SO₄; 25 mM MgSO₄; 1 % (v/v) Triton® X-100; 1 mg/ml BSA, 10 fmol plasmid, 100 pmol Primer L, 100 µmol Primer R, 200 µM dNTPs, 2.5 U PfuUltra DNA polymerase (Stratagene), ad 100 µl aqua dest. The following cycler protocol was used: 1' 94 °C, 25 cycles of 1' 94 °C, 1' 55 °C, 1.5' 72 °C, one cycle of 6' 72 °C. The 909 bp PCR products were purified using the MinElute PCR Purification Kit following the suppliers' instructions (Qiagen, Hilden).

For heteroduplex formation 2 µg (3.3 pmol) of each of the PCR products were mixed in 40 µl 10 mM Tris-Cl, pH 8.5, heated at 94°C for 5 min, gradually cooled down (0.04 °C/s) and incubated at 65 °C for 1 h and then again allowed to cool slowly (0.04 °C/s) down to 42 °C and incubated at this temperature for another h in order to reanneal strands and thereby produce heteroduplices. (94 °C 5' -> 65 °C 1h with 0.04 °C/s and 65 °C -> 42 °C 1h with 0.04 °C/s). The generated heteroduplex molecules contained 8 mismatches each (16 alltogether).

In order to generate single strand breaks, enzymes MutY and TDG were employed which specifically at mismatch sites remove the nucleobase and catalyze a single strand break leaving a deoxyribose-5-phosphate residue. Therefore, the heteroduplex DNA was incubated in 40 µl 10 mM HEPES_KOH, pH 7.4, 100 mM KCl, 10 mM EDTA with 8 U of E. coil MutY and TDG DNA glycosylases (Trevigen, Gaithersburg, MD) at 37°C (MutY) and 65°C (TDG) for 1 h at each temperature. Samples were purified using the MinElute PCR Purification Kit.

In order to remove the deoxyribose-5-phosphate from the 3' ends at the nicked abasic sites, the DNA was incubated with 0.05 U/µl E. coli Endonuclease IV (MBI Fermentas, St. Leon-Rot, Germany) in 50 mM Tris-acetate, pH 7.5; 50 mM KCl; 1 mM EDTA; 0.05% Triton® X-100. After incubation for 2 h at 37°C the proteins were removed by extraction with phenol/chloroform and the DNA was precipitated with ethanol.

In order to randomize at the mismatch positions, the single nucleotide-gap was filled with dITP. Therefore, the precipitated DNA was dissolved in 50 µl 50 mM Tris-Cl, pH 8.8; 10 mM MgCl₂; 100 mM KCI; 1.0 mM DTT; 10 % glycerol and incubated with 100 µM dITP and 8 U DNA polymerase beta at 37 °C for 1 h. Then the reaction mix was incubated with 0.1 U/µl T4 DNA ligase in 40 µM Tris-HCl, pH 7.8; 10 mM MgCl₂; 10 mM DTT, 0.5 mM ATP at 16 °C for 12 h. Samples were purified using the MinElute PCR Purification Kit. Then, the deoxyinosine-containing polynucleotides are used as templates in a polymerase extension reaction. Therefore, a PCR was performed by mixing 100 µl 75 mM Tris-HCl, pH 8.8, 20 mM (NH₄)₂SO₄, 2 mM MgCl₂; 0.01 % (v/v) Tween® 20, 0.8 pmol template, 100 pmol Primer L, 100 pmol Primer R, 200 µM dNTPs, 4 U Taq DNA polymerase and 1 U Pfu DNA polymerase (MBI- Fermentas). The following cycler protocol was used: 1' 94 °C, 20 cycles consisting of 1' 94 °C, 1' 55 °C, 2' 72 °C, one cycle 6' 72 °C. The resulting DNA fragments were purified using the MinElute PCR Purification Kit following the suppliers' instructions. The PCR fragments were digested with DraIII, ligated into a plasmid linearized with DraIII and transformed into E. coli XL-1 blue. Transformands were checked for carrying an insert of the expected length. The PCR products of ten positive transformands were purified using the MinElute PCR Purification Kit and analyzed by sequencing. Out of the ten randomly chosen sequences, one had the sequence of the mutant (SEQ ID NO:6) and the other nine had one or more positions mutated, with the majority (eight of nine) having one position mutated (from eight possible positions per gene).

## Claims

1. A method for randomizing polynucleotides at specific sites with no sequence related determination of differing sites needed which comprises the following steps:
(a) providing at least one polynucleotide having at least one differing site, whereby the differing sites define the starting points for randomization;
(b) generating at least one heteroduplex from the at least one polynucleotide of step (a);
(c) recognizing the resulting one or more mismatching site(s);
(d) selectively randomizing the polynucleotides at or in proximity to these mismatching sites.

2. The method of claim 1, wherein the heteroduplex of step (b) is derived from at least one starting single-strand polynucleotide or is a heteroduplex generated from at least two polynucleotides that differ in at least one site from each other.

3. The method of claim 1 or 2, wherein the polynucleotides of step (a) or their corresponding translational products are pre-selected with respect to their genotypic and/or phenotypic features.

4. The method of claim 1 to 3 wherein steps (a) to (d), steps (a) to (b) and/or steps (c) to (d) are carried out for multiple cycles before entering into the next step.

5. The method of claim 1 to 4 wherein the differing sites of the polynucleotides of step (a) leading to the mismatching sites of step (c) consist of one or more mutation(s), preferably the mutations comprise
(i) one or more nucleotide substitution(s),
(ii) one or more nucleotide insertion(s),
(iii) one or more nucleotide deletion(s), or
(iv) a combination of (i) to (iii).

6. The method of claim 1 to 5 which further comprises selection or screening for at least one selectively randomized polynucleotide or its corresponding translational products towards a desired property.

7. The method of claim 1 to 6 which is carried out cyclically.

8. The method of claim 1 to 7, wherein step (a) comprises providing variants of the polynucleotide sequence having at least one differing site and selectively randomizing the polynucleotide sequence at or in proximity to the differing site(s).

9. A method for randomizing polynucleotides at specific sites which comprises the following steps:
(a) providing polynucleotides that differ at one or more sites from each other, whereby these one or more differing sites specify the sites that are to be randomized;
(b) generating heteroduplexes from the polynucleotides provided in step (a) leading to mismatches at the one or more differing sites;
(c) removing at least one nucleobase at the one or more mismatches generated in step (b), by means of an agent that is able to specifically recognize mismatch sites thereby generating an abasic site at the one or more mismatches;
(d) separating the heteroduplex strands from each other; and
(e) synthesizing the counter strands using the single strands generated as templates, thereby randomizing the polynucleotides specifically at sites where the abasic sites were generated in step (c).

10. A method for randomizing polynucleotides at specific sites which comprises the following steps:
(a) providing polynucleotides that differ at one or more sites from each other, whereby these one or more differing sites specify the sites that are to be randomized;
(b) generating heteroduplices from the polynucleotides provided in step (a) leading to mismatches at the one or more sites;
(c) introducing single-strand nicks at one or more of the mismatches generated in step (b), by means of an agent that is able to specifically recognize mismatch sites;
(d) removing one or more nucleotides from the polynucleotide heteroduplex starting at the single-strand nicks generated in step (c);
(e) filling the one or more gaps produced in step (d) under conditions that lead to the incorporation of one or more mismatching nucleotides, thereby randomizing the polynucleotides at the specific sites.

11. A method for randomizing polynucleotides at specific sites which comprises the following steps:
(a) providing polynucleotides that differ at one or more sites from each other, whereby these one or more differing sites specify the sites that are to be randomized;
(b) generating heteroduplices from the polynucleotides provided in step (a) leading to mismatches at the one or more differing sites;
(c) introducing single-strand nicks at one or more of the mismatches generated in step (b), by means of an agent that is able to specifically recognize mismatch sites;
(d) removing one or more nucleotides from the polynucleotide heteroduplex starting at the single-strand nicks generated in step (c);
(e) filling the one or more gaps produced in step (d) at least in part with universal monomers, whereby universal monomers are characterized as being able to form basepairs alternatively with two or more of the four natural nucleobases;
(f) separating the heteroduplex strands from each other; and
(g) synthesizing the counter strands using the single strands generated in step
(f) as templates, thereby randomizing the polynucleotides specifically at sites where universal monomers were introduced in step (e).

12. The method according to claim 10 or 11, wherein
(i) the introduction of nicks in step (c) comprises the introduction of sole single-strand break in the phosphodiester backbone at the 3' or 5' side of the mismatching site, or the removal of the entire mismatch nucleotide, or the removal of several nucleotides at or around the mismatch site; and/or
(ii) the removal of nucleotides according to step (d) is either limited to several nucleotides to generate a single-strand region in proximity to the mismatch site, or is unrestricted to generate a gap from the mismatch position to the end of the polynucleotide; and/or
(iii) the removal of one or more nucleotides according to step (d) and with filling of the gap according to step (e) are carried out in parallel by means of a standard polymerase, a polymerase having 5'-3' exonuclease or strand displacement activity; and/or
(iv) the filling of the gap according to step (e) is carried out at least in part by use of oligonucleotides and a ligase enzyme.

13. The method according to any one of claims 10 to 12, wherein the filling of the gap according to step (e) is carried out with a polymerase and
(i) a mixture of 3 of the 4 standard nucleotides (dATP, dTTP, dGTP, dCTG), or
(ii) separately with different compositions of mixtures of 3 nucleotides (dATP, dTTP, dGTP, dCTG), or
(iii) separately with one of the 4 standard nucleotides (dATP, dTTP, dGTP, dCTG) provided in each reaction
with preferably the separately filled gaps according to step (e) are pooled afterwards.

14. The method according to any one of claims 10 to 13, wherein the filling of the gap according to step (e) is carried out with a polymerase under highly mutagenic conditions or with a low-fidelity polymerase having a high error rate.

15. The method according to any one of claims 10 to 13, wherein the filling of the gap according to step (e) is carried out with a polymerase and dITP instead of dATP, dTTP, dGTP, dCTG or a mixture of dITP and dATP, dTTP, dGTP, dCTG in same or different concentrations.

16. A method for optimizing a polynucleotide sequence with no sequence related determination of differing sites needed, comprising
(a) providing variants of a polynucleotide sequence that differ at one or more site(s) from each other;
(b) generating at least one heteroduplex from the variants of a polynucleotide sequence provided in step (a);
(c) recognizing the differing sites within the heteroduplices;
(d) randomizing the polynucleotide sequence specifically at those sites at which these variants differ from each other;
(e) selecting or screening the randomized pool of polynucleotides for desired properties.

17. A method for optimizing a polynucleotide towards desired properties of its translational product with no sequence related determination of differing sites needed which comprises
(a) introducing stochastically random mutations into polynucleotides;
(b) selecting or screening the population of polynucleotides generated in step (a);
(c) isolating those polynucleotides which encode gene products with improved characteristics;
(d) generating at least one heteroduplex from the polynucleotides isolated in step (c) that differ at one or more site(s) from each other,
(e) recognizing the resulting mismatching sites;
(f) selectively randomizing the polynucleotides at or in proximity to those mismatching sites;
(g) selecting or screening the population of polynucleotides generated in step (f);
(h) isolating those polynucleotides which encode gene products with further improved characteristics,
wherein in the above method steps (a) to (c) and/or steps (d) to (h), and/or steps (a) to (h) are preferably repeated iteratively.

## Patentansprüche

1. Verfahren zur Randomisierung von Polynukleotiden an bestimmten Stellen, wobei eine sequenzbezogene Ermittlung abweichender Stellen nicht notwendig ist, umfassend die folgenden Schritte:
(a) Bereitstellung mindestens eines Polynukleotids mit mindestens einer abweichenden Stelle, wobei die abweichenden Stellen die Startpunkte der Randomisierung darstellen;
(b) Herstellung mindestens einer Heteroduplex aus dem mindestens einen Polynukleotid aus Schritt (a);
(c) Erkennung der resultierenden einen oder mehreren Fehlpaarungsstelle(n);
(d) selektive Randomisierung der Polynukleotide an oder in der Nähe dieser Fehlpaarungsstellen.

2. Verfahren nach Anspruch 1, wobei die Heteroduplex aus Schritt (b) von mindestens einem einzelsträngigen Anfangspolynukleotid abgeleitet wird, oder eine Heteroduplex ist, die aus mindestens zwei an mindestens einer Stelle voneinander abweichenden Polynukleotiden gebildet wird.

3. Verfahren nach Anspruch 1 oder 2, wobei die Polynukleotide aus Schritt (a) oder ihre korrespondierenden Translationsprodukte hinsichtlich ihrer genotypischen und/oder phänotypischen Eigenschaften vorselektiert werden.

4. Verfahren nach Anspruch 1 bis 3, wobei die Schritte (a) bis (d), die Schritte (a) bis (b) und/oder die Schritte (c) bis (d) über mehrere Zyklen durchgeführt werden, bevor mit dem nächsten Schritt begonnen wird.

5. Verfahren nach Anspruch 1 bis 4, wobei die abweichenden Stellen der Polynukleotide aus Schritt (a), die zu den Fehlpaarungsstellen in Schritt (c) führen, aus einer oder mehreren Mutation(en) bestehen, vorzugsweise umfassen die Mutationen
(i) eine oder mehrere Nukleotidsubstitution(en),
(ii) eine oder mehrere Nukleotidinsertion(en),
(iii) eine oder mehrere Nukleotiddeletion(en), oder
(iv) eine Kombination von (i) bis (iii).

6. Verfahren nach Anspruch 1 bis 5, welches ferner die Selektion oder das Durchmustern von mindestens einem selektiv randomisierten Polynukleotid oder ihren korrespondierenden Translationsprodukten auf eine gewünschte Eigenschaft umfasst.

7. Verfahren nach Anspruch 1 bis 6, welches zyklisch durchgeführt wird.

8. Verfahren nach Anspruch 1 bis 7, wobei Schritt (a) die Bereitstellung von Varianten der Polynukleotidsequenzen mit mindestens einer abweichenden Stelle und selektiver Randomisierung der Polynukleotidsequenz an oder in der Nähe zu der/den abweichenden Stelle(n) umfasst.

9. Verfahren zur Randomisierung von Polynukleotiden an bestimmten Stellen, welches die folgenden Schritte umfasst:
(a) Bereitstellung von Polynukleotiden, die an einer oder mehreren Stellen voneinander abweichen, wobei diese eine oder mehreren Stelle(n) die Stellen bestimmen an denen randomisiert wird;
(b) Herstellung von Heteroduplices aus den in Schritt (a) bereitgestellten Polynukleotiden, die zur Fehlpaarung an einer oder mehreren abweichenden Stelle(n) führen;
(c) Entfernung mindestens einer Nukleobase an einer oder mehreren in Schritt (b) hergestellten Fehlpaarung(en) mittels eines Agens, das in der Lage ist Fehlpaarungsstellen spezifisch zu erkennen und dabei eine nicht-basische Stelle an einer oder mehreren Fehlpaarung(en) zu erzeugen;
(d) Trennung der Heteroduplexstränge voneinander; und
(e) Synthetisierung der Gegenstränge unter Verwendung der hergestellten Einzelstränge als Matrizen, wobei die Polynukleotide genau an den Stellen randomisiert werden, an denen die nicht-basischen Stellen in Schritt (c) erzeugt wurden.

10. Verfahren zur Randomisierung von Polynukleotiden an bestimmten Stellen, umfassend die folgenden Schritte:
(a) Bereitstellung von Polynukleotiden, die an einer oder mehreren Stellen voneinander abweichen, wobei diese eine oder mehreren Stelle(n) die Stellen bestimmen, an denen randomisiert wird;
(b) Herstellung von Heteroduplices aus den in Schritt (a) bereitgestellten Polynukleotiden, die zu Fehlpaarungen an einer oder mehreren Stelle(n) führen;
(c) Einfügen von Einzelstrangbrüchen an einer oder mehreren in Schritt (b) hergestellten Fehlpaarungsstelle(n) mittels eines Agens, das in der Lage ist Fehlpaarungsstellen spezifisch zu erkennen;
(d) Entfernen eines oder mehrerer Nukleotid(e) aus der Polynukleotidheteroduplex beginnend an dem in Schritt (c) erzeugten Einzelstrangbruch;
(e) Auffüllen der einen oder mehreren in Schritt (d) erzeugten Lücke(n) unter Bedingungen, die zum Einbau eines oder mehrerer fehlgepaarter Nukleotide führen und dadurch die Polynukleotide an den bestimmten Stellen randomisieren.

11. Verfahren zur Randomisierung von Polynukleotiden an bestimmten Stellen, umfassend die folgenden Schritte:
(a) Bereitstellung von Polynukleotiden, die an einer oder mehreren Stellen voneinander abweichen, wobei diese eine oder mehreren Stelle(n) die Stellen bestimmen an denen randomisiert wird;
(b) Herstellung von Heteroduplices aus den in Schritt (a) bereitgestellten Polynukleotiden, die zu Fehlpaarungen an der oder den abweichenden Stelle(n) führen;
(c) Einfügen von Einzelstrangbrüchen an einer oder mehreren in Schritt (b) erzeugten Fehlpaarung(en) mittels eines Agens, das in der Lage ist Fehlpaarungsstellen spezifisch zu erkennen;
(d) Entfernen eines oder mehrerer Nukleotid(e) aus der Polynukleotidheteroduplex beginnend an dem in Schritt (c) hergestellten Einzelstrangbruch;
(e) zumindest teilweises Auffüllen der einen oder mehreren in Schritt (d) hergestellten Lücke(n) mit universellen Monomeren, wobei sich die universellen Monomere dadurch auszeichnen, dass sie in der Lage sind, mit zwei oder wahlweise mehr der natürlichen Nukleobasen Basenpaare zu bilden;
(f) Trennung der Heteroduplexstränge voneinander; und
(g) Synthetisierung der Gegenstränge unter Verwendung der hergestellten Einzelstränge als Matrizen, wobei die Polynukleotide spezifisch an den Stellen randomisiert werden, an denen die universellen Monomere in Schritt (c) eingefügt wurden.

12. Verfahren gemäß Anspruch 10 oder 11, wobei
(i) das Einfügen der Brüche in Schritt (c) das Einfügen eines einzigen Einzelstrangbruchs in dem Phosphodiester Rückgrat an der 3' oder 5' Seite der Fehlpaarungsstelle, oder das Entfernen des gesamten fehigepaarten Nukleotids, oder das Entfernen mehrerer Nukleotide an oder um die Fehlpaarungsstelle, umfasst; und/oder
(ii) das Entfernen der Nukleotide gemäß Schritt (d) entweder auf mehrere Nukleotide zur Herstellung eines Einzelstrangbereiches in Nachbarschaft zu der Fehlpaarungsstelle begrenzt ist, oder durch Erzeugen eine Lücke von der Fehlpaarungsposition bis zum Ende des Polynukleotids nicht begrenzt ist; und/oder
(iii) das Entfernen von einem oder mehreren Nukleotid(en) gemäß Schritt (d) und das Auffüllen der Lücke gemäß Schritt (e) parallel mittels einer Standardpolymerase, einer Polymerase mit 5'-3' Exonuklease- oder Strangverdrängungsaktivität, durchgeführt wird; und/oder
(iv) das Auffüllen der Lücke gemäß Schritt (e) zumindest teilweise unter Verwendung von Oligonukleotiden und einem Ligaseenzym durchgeführt wird.

13. Verfahren gemäß einem der Ansprüche 10 bis 12, wobei das Auffüllen der Lücke gemäß Schritt (e) mit einer Polymerase und
(i) einer Zusammensetzung aus 3 der 4 Standardnukleotide (dATP, dTTP, dGTP, dCTG), oder
(ii) getrennt mit verschiedenen Zusammensetzungen an Mischungen aus 3 Nukleotiden (dATP, dTTP, dGTP, dCTG), oder
(iii) getrennt mit einem der 4 für jede Reaktion bereitgestellten Standardnukleotide (dATP, dTTP, dGTP, dCTG),
durchgeführt wird, wobei vorzugsweise die getrennt aufgefüllten Lücken gemäß Schritt (e) anschließend zusammengefasst werden.

14. Verfahren gemäß einem der Ansprüche 10 bis 13, wobei das Auffüllen der Lücke gemäß Schritt (e) mit einer Polymerase unter hoch mutagenen Bedingungen oder mit einer Polymerase geringer Genauigkeit mit einer hohen Fehlerrate durchgeführt wird.

15. Verfahren gemäß einem der Amsprüche 10 bis 13, wobei das Auffüllen der Lücke gemäß Schritt (e) mit einer Polymerase und dITP anstelle von dATP, dTTP, dGTP, dCTG oder einer Mischung von dITP und dATP, dTTP, dGTP, dCTG in gleichen oder verschiedenen Konzentrationen durchgeführt wird.

16. Verfahren zur Optimierung einer Polynukleotidsequenz bei dem die sequenzbezogene Ermittlung abweichender Stellen nicht notwendig ist, umfassend
(a) Bereitstellung von Varianten einer Polynukleotidsequenz, die an einer oder mehreren Stelle(n) voneinander abweichen;
(b) Herstellung mindestens einer Heteroduplex aus dem mindestens einem Polynukleotid aus Schritt (a);
(c) Erkennen der abweichenden Stellen innerhalb der Heteroduplices;
(d) Randomisierung der Polynukleotidsequenzen spezifisch an jenen Stellen, an denen diese Varianten sich voneinander abweichen;
(e) Selektieren und Durchmustern des randomisierten Pools an Polynukleotiden auf gewünschte Eigenschaften.

17. Verfahren zur Optimierung von Polynukleotiden hin zü gewünschten Eigenschaften ihrer Translationsprodukte bei dem die sequenzbezogene Ermittlung abweichender Stellen nicht notwendig ist, umfassend
(a) stochastisches Einfügen zufälliger Mutationen in Polynukleotide;
(b) Selektieren und Durchmustern der in Schritt (a) hergestellten Population an Polynukleotiden;
(c) Isolierung der Polynukleotide, die Genprodukte mit verbesserten Eigenschaften kodieren;
(d) Herstellung mindestens einer Heteroduplex aus den in Schritt (c) isolierten Polynukleotiden, die an einer oder mehreren Stelle(n) voneinander abweichen;
(e) Erkennen der resultierenden Fehlpaarungsstellen;
(f) selektives Randomisieren der Polynukleotide an oder in der Nähe dieser Fehlpaarungsstellen;
(g) Selektieren und Durchmustern der in Schritt (f) hergestellten Population an Polynukleotiden;
(h) Isolierung der Polynukleotide, die Genprodukte mit weiteren verbesserten Eigenschaften kodieren,
wobei die obigen Verfahrensschritte (a) bis (c) und/oder Schritte (d) bis (h), und/oder Schritte (a) bis (h) vorzugsweise iterativ wiederholt werden.

## Revendications

1. Une méthode pour rendre aléatoire des polynucléotides à des sites spécifiques sans qu'il soit requis la détermination des différents sites liés à séquence qui comprend les étapes suivantes :
(a) la fourniture d'au moins un polynucléotide ayant au moins un site différent, dans lequel les différents sites définissent les points de départ de l'aération;
(b) la génération d'au moins un hétéroduplex à partir du au moins un nucléotide de l'étape (a) ;
(c) la reconnaissance d'un ou plus sites mal apparié(s);
(d) effectuer l'aléation sélective de polynucléotides sur ou dans la proximité de ces sites mal appariés;

2. La méthode de la revendication 1, dans laquelle l'hétéroduplex de l'étape (b) est dérivée du au moins un polynucléotide à un brin de départ ou est un hétéroduplex généré à partir de au moins deux polynucléotides qui diffèrent en au moins un site l'un par rapport à l'autre.

3. La méthode de la revendication 1 ou 2, dans laquelle le polynucléotide de l'étape (a) ou leurs produits de traduction correspondante sont pré-sélectionnés par rapport à leurs caractéristiques génotypiques et/ou phénotypiques.

4. La méthode des revendications 1 à 3, dans laquelle les étapes (a) à (d), les étapes (a) à (b) et/ou les étapes (c) à (d) sont effectuées pendant des cycles multiples avant d'effectuer l'étape suivante.

5. La méthode des revendications 1 0 4, dans laquelle les différents sites des polynucléotides de l'étape (a) générant les sites malappérés ?? de l'étape (c) consistent en un ou plusieurs mutations, de préférence les mutations comprennent :
(i) une ou plusieurs substitutions nucléotides,
(ii) une ou plusieurs insertions nucléotides,
(iii) une ou plusieurs suppressions nucléotides, ou
(iv) une combinaison de (i) à (iii).

6. La méthode des revendications 1 à 5, qui comprend en outre la sélection ou la sélection d'au moins un polynucléotide rendu aléatoire ou son produit de traduction correspondante afin d'obtenir une propriété désirée.

7. La méthode des revendications 1 à 6, qui est effectuée d'une manière cyclique.

8. La méthode des revendications 1 à 7, dans laquelle l'étape (a) comprend la fourniture de variantes de la séquence de polynucléotides ayant au moins un site différent et l'aléation sélective de la séquence de polynucléotides sur ou à proximité du ou des sites différents.

9. Une méthode pour aléer des polynucléotides à des sites spécifiques qui comprennent les étapes suivantes :
(a) fournir des polynucléotides qui sont différents à un ou plusieurs sites les uns des autres, dans lesquels ces un ou plusieurs sites différents spécifient les sites qui doivent être aléés ;
(b) la génération d'hétéroduplex à partir de polynucléotides fournis à l'étape (a) générant des nouveaux appariements à un ou plus des sites différents.
(c) la suppression d'au moins une nucléobase à une ou plusieurs des mauvais appariements générés à l'étape (b), par le moyen d'un agent qui est capable de reconnaître spécifiquement des sites mal appariés, par cela générant un site abasique à l'un ou plusieurs des mauvaix appariements;
(d) la séparation des brins d'hétéroduplex les uns par rapport aux autres ; et
(e) la synthèse des brins complémentaires utilisant les brins seuls générés comme matrices, par cela aléant les polynucléotides spécifiquement aux sites où les sites abasiques étant générés à l'étape (c).

10. Une méthode pour aléer des polynucléotides à des sites spécifiques qui comprend les étapes suivantes:
(a) la fourniture de polynucléotides qui sont différents les uns des autres à un ou plusieurs sites, dans lesquels ces un ou plusieurs sites différents spécifient les sites qui doivent être aléés ;
(b) la génération d'hétéroduplex à partir des polynucléotides fournis à l'étape (a) générant des mauvais appariements à un ou plusieurs sites ;
(c) l'introduction de cassures sur un seul brin à un ou plusieurs des mauvais appariements générés à l'étape (b), par l'utilisation d'un agent qui est capable spécifiquement de reconnaître les sites mal appariés ;
(d) la suppression d'un ou de plusieurs nucléotides de l'hétéroduplex du polynucléotide commençant aux cassures d'un seul brin généré à l'étape (c);
(e) le remplissage d'un ou de plusieurs espaces produits à l'étape (d) dans des conditions qui conduisent à l'incorporation d'un ou de plusieurs nucléotides mal appariés, par cela aléant des polynucléotides à des sites spécifiques.

11. Une méthode pour aléer des polynucléotides à des sites spécifiques qui comprend les étapes suivantes :
(a) la fourniture de polynucléotides qui différent les uns des autres à un ou plusieurs sites, dans lesquels ces un ou plusieurs sites spécifient les sites qui doivent être aléés ;
(b) la génération d'hétéroduplex à partir des polynucléotides fournis à l'étape (a) générant des mauvais appariements à un ou plusieurs des sites différents ;
(c) l'introduction de cassures à un brin à un ou plusieurs des mauvais appariements générés à l'étape (b), par l'utilisation d'un agent qui est capable spécifiquement de reconnaître les sites mal appariés ;
(d) la suppression d'un ou de plusieurs nucléotides de l'hétéroduplex polynucléotide commençant aux cassures sur un seul brin générées à l'étape (c) ;
(e) le remplissage d'un ou de plusieurs espaces produits à l'étape (d) au moins en partie avec des monomères universels, dans lesquels les monomères universels sont **caractérisés en ce qu'**ils sont capables de former des paires de bases alternativement avec deux ou plus des quatre nucléobases naturelles;
(f) la séparation des brins de l'hétéroduplex les uns des autres ; et
(g) la synthèse des brins opposés en utilisant les brins seuls générés à l'étape (f) comme matrices, par cela aléant les polynucléotides spécifiquement aux sites où les monomères universels ont été introduits à l'étape (e).

12. La méthode selon la revendication 10 ou 11, dans laquelle:
(i) l'introduction des cassures à l'étape (c) comprend l'introduction de cassure sur un seul brin dans le squelette phosphodiester sur le côté 3' ou 5' du site mal apparié, ou la suppression du nucléotide entier mal apparié, ou la suppression de plusieurs nucléotides sur ou autour du site mal apparié ; et/ou
(ii) la suppression des nucléotides selon l'étape (d) est soit limitée à quelques nucléotides pour générer une région ayant un seul brin à proximité du site mal apparié, ou n'est pas restreint pour générer un espace de la position mal apparié à la fin du polynucléotide ; et/ou
(iii) la suppression d'un ou de plusieurs nucléotides selon l'étape (d) et avec le remplissage de l'espace selon l'étape (e) qui sont effectués en parallèle par l'utilisation d'une polymérase standard, une polymérase ayant une activité exonucléase 5'-3' ou une activité de déplacement de brin ; et/ou
(iv) le remplissage de l'espace selon l'étape (e) est effectué au moins en partie par l'utilisation d'oligonucléotides et d'une enzyme ligase.

13. La méthode selon l'une quelconque des revendications 10 à 12, dans laquelle le remplissage de l'espace selon l'étape (e) est effectué avec une polymérase et
(i) un mélange de 3 des 4 nucléotides standard (dATP, dTTP, dGTP, dCTG), ou
(ii) séparément avec différentes compositions de mélanges de 3 nucléotides (dATP, dTTP, dGTP, dCTG), ou
(iii) séparément avec un des quatre nucléotides standard (dATP, dTTP, dGTP, dCTG) fournis dans chaque réaction
de préférence avec les espaces remplis séparément selon l'étape (e) sont assemblés après.

14. La méthode selon l'une quelconque des revendications 10 à 13, dans laquelle le remplissage de l'espace selon l'étape (e) est effectué avec une polymérase dans des conditions hautement mutagènes et avec une polymérase ayant un fidélité faible et ayant un taux d'erreur élevé.

15. La méthode selon l'une quelconque des revendications 10 à 13, dans laquelle le remplissage de l'espace selon l'étape (e) est effectué avec une polymérase et dTTP à la place de dATP, dTTP, dGTP, dCTG ou un mélange de dTTP et dATP, dTTP, dGTP, dCTG dans les mêmes ou des concentrations différentes.

16. Une méthode pour optimiser une séquence de polynucléotide sans détermination des sites différents requis liés à la séquence, comprenant :
(a) la fourniture de variantes d'une séquence de polynucléotides qui diffèrent à un ou plusieurs sites les uns des autres ;
(b) la génération d'au moins un hétéroduplex à partir de variantes de la séquence de polynucléotides fournie à l'étape (a) ;
(c) la reconnaissance de différents sites dans les hétéroduplex ;
(d) l'aléation de la séquence de polynucléotides spécifiquement aux sites auxquels ces variantes différent les unes des autres ;
(e) la sélection ou la détermination de l'échantillon aléé de polynucléotides pour des propriétés désirées.

17. Une méthode pour optimiser un polynucléotide vers des propriétés désirées de son produit de traduction sans détermination des sites différents requis liés à la séquence qui comprend :
(a) l'introduction de mutations aléatoires stochastiques dans des polynucléotides;
(b) la sélection ou la détermination de la population de polynucléotides générés à l'étape (a) ;
(c) l'isolation de polynucléotides qui encodent des produits de gène avec des caractéristiques améliorées;
(d) la génération d'au moins un hétéroduplex à partir de polynucléotides isolés à l'étape (c) qui différent à un ou plusieurs sites les uns des autres ;
(e) la reconnaissance des sites mal appariés en résultant;
(f) l'aléation sélective de polynucléotides sur ou à proximité de ces sites mal appariés ;
(g) la sélection ou la détermination de la population de polynucléotides générés à l'étape (f);
(h) l'isolation de ces polynucléotides qui encodent des produits de gène avec des caractéristiques améliorées,
dans lesquels les étapes des méthodes (a) à (c) et/ou étapes (d) à (h) et/ou étapes (a) à (h) sont de préférence répétées de manière itérative.


### SEQUENCE LISTING

<110> DIREVO Biotech AG
<120> Method for the Selective Combinatorial Randomization of Polynucleotides
<130> 031969wo/JH/ml
<140>
   <141>
<160> 17
<170> PatentIn Ver. 2.1
<210> 1
   <211> 116
   <212> DNA
   <213> Artificial Sequence
<220>
   <223> Description of Artificial Sequence: Polynucleotide 1
<400> 1
   cgttgcatat gtggaagaag atcatattgc acatgaatat gcacagagtg ttccttatgg 60
   catttctcaa attaaagcgc cggctcttca ctctcaaggc tacacaggct ctaacg     116
<210> 2
   <211> 116
   <212> DNA
   <213> Artificial Sequence
<220>
   <223> Description of Artificial Sequence: Polynucleotide 2
<400> 2
   cgttgcatat gtggaagaag atcatattgc acatgaatat gcacagagtg ctccttatgg 60
   catttctcaa attaaagcgc cggctcttca ctctcaaggc tacacaggct ctaacg     116
<210> 3
   <211> 419
   <212> DNA
   <213> Artificial Sequence
<220>
   <223> Description of Artificial Sequence: Polynucleotide
   3
<400> 3
   cgttgcatat gtggaagaag atcatattgc acatgaatat gcacagagtg ttccttatgg 60
   catttctcaa attaaagcgc cggctcttca ctctcaaggc tacacaggct ctaacgtaaa 120
   agtagctgtt atcgacagcg gaattgactc ttctcatcct gacttaaacg tcagaggcgg 180
   agcaagcttc gtaccttctg aaacaaaccc ataccaggac ggcagttctc acggtacgca 240
   tgtagccggt acgattgccg ctcttaataa ctcaatcggt gttctgggcg tagcgccaag 300
   cgcatcatta tatgcagtaa aagtgcttga ttcaacagga agcggccaat atagctggat 360
   tattaacggc attgagtggg ccatttccaa caatatggat gttatcaaca tgagccttg  419
<210> 4
   <211> 419
   <212> DNA
   <213> Artificial Sequence
<220>
   <223> Description of Artificial Sequence: Polynucleotide
   4
<400> 4
   cgttgcatat gtggaagaag atcatattgc acatgaatat gcacagagtg ctccttatgg 60
   catttctcaa attaaagcgc cggctcttca ctctcaaggc tacacaggct ctaacgtaaa 120
   agtagctgtt atcgacagcg gaattgactc ttctcatcct gacttaaacg taagaggcgg 180
   agcaagcttc gtaccttctg atacaaaccc ataccaggac agcagttctc acggtacgca 240
   tgtagccggt acgattgctg ctcttaataa ctcaatcggt gttctgggcg tagcgccaag 300
   cgcatcatta tatgcagtaa aagtgcttga ttcaacagga agcggccgtt atagctggat 360
   tattaacggc attgagtggg ccatttccaa caatatggat gttatcaaca tgagccttg  419
<210> 5
   <211> 27
   <212> DNA
   <213> Artificial Sequence
<220>
   <223> Description of Artificial Sequence: Oligonucleotide 1
<220>
   <221> modified_base
   <222> (17)
   <223> abasic deoxynucleotide
<400> 5
   gaatatgcac agagtgntcc ttatggc                                     27
<210> 6
   <211> 27
   <212> DNA
   <213> Artificial Sequence
<220>
   <223> Description of Artificial Sequence: Oligonucleotide 2
<400> 6
   gccataagga gcactctgtg catattc                                     27
<210> 7
   <211> 27
   <212> DNA
   <213> Artificial Sequence
<220>
   <223> Description of Artificial Sequence: Primer 23
<400> 7
   gccataagga gcactctgtg catattc                                     27
<210> 8
   <211> 33
   <212> DNA
   <213> Artificial Sequence
<220>
   <223> Description of Artificial Sequence: Primer 11
<400> 8
   gcacatgaat atgcacagag tgttccttat ggc 33
<210> 9
   <211> 65
   <212> DNA
   <213> Artificial Sequence
<220>
   <223> Description of Artificial Sequence: Template 31
<220>
   <221> misc_feature
   <222> (34)
   <223> n= A, T, C or G
<400> 9
   gccataagga acactctgtg catattcatg tgcnatatga tcttcttcca catatgcaac 60
   gctcg 65
<210> 10
   <211> 32
   <212> DNA
   <213> Artificial Sequence
<220>
   <223> Description of Artificial Sequence: Oligonucleotide 3
<220>
   <221> modified_base
   <222> (32)
   <223> i
<400> 10
   cgagcgttgc atatgtggaa gaagatcata tn 32
<210> 11
   <211> 33
   <212> DNA
   <213> Artificial Sequence
<220>
   <223> Description of Artificial Sequence: Oligonucleotide 4
<400> 11
   gcacatgaat atgcacagag tgttccttat ggc 33
<210> 12
   <211> 27
   <212> DNA
   <213> Artificial Sequence
<220>
<223> Description of Artificial Sequence: Primer 1
<220>
   <221> modified_base
   <222> (14)
   <223> i
<400> 12
   gatcatattg cacntgcata tgcacag 27
<210> 13
   <211> 22
   <212> DNA
   <213> Artificial Sequence
<220>
   <223> Description of Artificial Sequence: Primer 2
<400> 13
   caaggctcat gttgataaca tc 22
<210> 14
   <211> 909
   <212> DNA
   <213> Artificial Sequence
<220>
   <223> Description of Artificial Sequence: Polynucleotide 14
<400> 14
   cgttgcatat gtggaagaag atcatattgc acatgaatat gcacagagtg ttccttatgg 60
   catttctcaa attaaagcgc cggctcttca ctctcaaggc tacacaggct ctaacgtaaa 120
   agtagctgtt atcgacagcg gaattgactc ttctcatcct gacttaaacg tcagaggcgg 180
   agcaagcttc gtaccttctg aaacaaaccc ataccaggac ggcagttctc acggtacgca 240
   tgtagccggt acgattgccg ctcttaataa ctcaatcggt gttctgggcg tagcgccaag 300
   cgcatcatta tatgcagtaa aagtgcttga ttcaacagga agcggccaat atagctggat 360
   tattaacggc attgagtggg ccatttccaa caatatggat gttatcaaca tgagccttgg 420
   cggacctact ggttctacag cgctgaaaac agtcgttgac aaagccgttt ccagcggtat 480
   cgtcgttgct gccgcagccg gaaacgaagg ttcatccgga agcacaagca cagtcggcta 540
   ccctgcaaaa tatccttcta ctattgcagt aggtgcggta aacagcagca accaaagagc 600
   ttcattctcc agcgcaggtt ctgagcttga tgtgatggct cctggcgtgt ccatccaaag 660
   cacacttcct ggaggcactt acggcgctta taacggaacg tccatggcga ctcctcacgt 720
   tgccggagca gcagcgttaa ttctttctaa gcacccgact tggacaaacg cgcaagtccg 780
   tgatcgttta gaaagcactg caacatatct tggaaactct ttctactatg gaaaagggtt 840
   aatcaacgta caagcagctg cacaataaca ctaggtgtaa aaagaagcag gttcctccat 900
   acctgcttc 909
<210> 15
   <211> 909
   <212> DNA
   <213> Artificial Sequence
<220>
   <223> Description of Artificial Sequence: Polynucleotide 15
<400> 15
   cgttgcatat gtggaagaag atcatattgc acatgaatat gcacagagtg ttccttatgg 60
   catttctcaa attaaagcgc cggctcttca ctctcaaggc tacacaggct ctaacgtaaa 120
   agtagctgtt atcgacagcg gaattgactc ttctcatcct gacttaaacg taagaggcgg 180
   agcaagcttc gtaccttctg atacaaaccc ataccaggac ggcagttctc acggtacgca 240
   tgtagccggt acgattgccg ctcttaataa ctcgatcggt gttctgggcg tagcgccaag 300
   cgcatcatta tatgcagtaa aagtgcttga ttcaacagga agcggccgtt atagctggat 360
   tattaacggc attgagtggg ccatttccaa caatatggat gttatcaaca tgagccttgg 420
   cggccctact ggttctaaag cgctgaaaac agtcgttgac aaagccgttt ccagcggtat 480
   tgtcgttgct gccgcagccg gaaacgcagg ttcatccgga agcacaagca cagtcggcta 540
   ccctgcaaaa tatccttcta ctattgcagt aggtgcggta aacagcagca accaaagagc 600
   ttcattctcc agcgcaggtt ccgagcttga tgtgatggct cctggcgtgt ccatccaaag 660
   cacacttcct ggaggcactt acggcgctca taacggaacg tccatggcga ctcctcacgt 720
   tgccggagca gcagcgttaa ttctttctaa gcacccgact tggacaaacg cgcaagtccg 780
   tgatcgttta gaaagcactg caacatatct tggtaactct ttctactatg gaaaagggtt 840
   aatcaacgta caagcagctg cacaataaca ctaggtgtaa aaagaagcag gttcctccat 900
   acctgcttc 909
<210> 16
   <211> 23
   <212> DNA
   <213> Artificial Sequence
<220>
   <223> Description of Artificial Sequence: Primer L
<400> 16
   cgttgcatat gtggaagaag atc                                                23
<210> 17
   <211> 19
   <212> DNA
   <213> Artificial Sequence
<220>
   <223> Description of Artificial Sequence: Primer R
<400> 17
   gaagcaggta tggaggaac                                                     19